# EUROPEAN PATENT SPECIFICATION

(11) **EP 3 753 926 B1**
(45) Date of publication and mention of the grant of the patent: **09.10.2024**
(21) Application number: 19754006.5
(22) Date of filing: 12.02.2019
(51) Int. Cl.: C07D 215/12, C07D 215/18, C07D 405/04, C07D 401/04, C07D 471/10, A61K 31/47, A61K 31/4709, A61P 25/00, A61P 25/22, A61P 25/24, A61P 25/28, A61P 27/02, A61P 31/18, A61P 35/00, A61P 37/00

(54) **SPIRO COMPOUND AS INDOLEAMINE-2,3-DIOXYGENASE INHIBITOR**
SPIROVERBINDUNG ALS INDOLAMIN-2,3-DIOXYGENASE-INHIBITOR
COMPOSÉ SPIRO UTILISÉ EN TANT QU'INHIBITEUR DE L'INDOLÉAMINE-2,3-DIOXYGÉNASE

(30) Priority: 13.02.2018 CN 201810150080
(43) Date of publication of application: 23.12.2020
(73) Proprietor: Shanghai Institute of Organic Chemistry, Chinese Academy of Sciences, Shanghai 200032 (CN)
(72) Inventor: WANG, Zhaoyin, Shanghai 200032 (CN); GUO, Wei, Shanghai 200032 (CN); CHAI, Yongshuai, Shanghai 200032 (CN)
(74) Representative: dompatent von Kreisler Selting Werner - Partnerschaft von Patent- und Rechtsanwälten mbB
(86) International application number: PCT/CN2019/074855
(87) International publication number: WO 2019/158051

(56) References cited:
- WO-A1-2017/192840
- WO-A1-2019/087028
- WO-A2-2019/078968
- CN-A- 101 312 976
- CN-A- 105 732 636
- CN-A- 111 471 010

## Description

### Technical field

The invention belongs to the technical field of pharmaceutical chemistry, in particular relates to an IDO inhibitor containing a spiro structure according to claim 1.

### Background technology

Indoleamine-2, 3-dioxygenase (IDO) is a monomeric enzyme containing heme discovered by Hayaishi group in 1967. The cDNA encoded protein consists of 403 amino acids at a molecular weight of 45 kDa, which is a rate-limiting enzyme in the catabolism of the tryptophan-kynurenine and widely expressed in many mammalian tissues. In tumor cells, IDO often plays an important role in inducing tumor microenvironment immune tolerance, whose tryptophan (TRP) - kynurenine (KYN) metabolic pathway is involved in the tumor immune escape; IDO also plays an important role in inducing tumor microenvironment immune tolerance.

Tryptophan, as one of the most important essential amino acids in mammals, needs to be taken massively from food to maintain cell activation and proliferation as well as the synthesis of protein and some neurotransmitters, whose deficiency, therefore, can result in the dysfunction of some important cells. IDO can catalyze the conversion of tryptophan to N'-formyl-L-Kynurenine in vivo and degrade the content of tryptophan, which results in the deficiency of tryptophan in vivo and leads to the occurrence of tumors. However, immunohistology suggests that the kynurenine pathway can lead to the increase of quinolinic acid, an excitotoxin as well as many serious human diseases such as Alzheimer.

There are two kinds of tryptophan rate-limiting enzymes in mammals: Tryptophan dioxygenase (TDO) and IDO. In 1937, Kotake et al. purified the protein from rabbit intestines and found that TDO is mainly expressed in mammalian liver for the first time. So far, it has not been found yet that TDO is closely correlated with the immune system. TDO can catalyze the kynurenine pathway and convert tryptophan to N'-formyl-L-Kynurenine. In 1978, the enzyme purified from rabbit intestines was identified as a dioxygenase (IDO) containing heme. IDO is the only enzyme that can catalyze the oxidative cleavage of indoles in tryptophan molecules and prolong the catabolism of kynurenine pathway in addition to the liver. IDO is usually expressed in organs with more mucous membranes, such as lung, small intestine, large intestine, rectum, spleen, kidney, stomach and brain. In such special/pathological conditions as pregnancy, chronic infection, organ transplantation and tumor, the expression of IDO will be significantly increased, involved in the local immunosuppression.

Studies suggested that IDO can inhibit local T cell immune response in the tumor microenvironment in the following ways: Tryptophan depletion, toxic metabolism and induction of regulatory T cell proliferation. Frequently, it is overexpressed in tumors, consuming local tryptophan and producing a great number of metabolites such as kynurenine. In fact, in the condition of culture without tryptophan or kynurenine, T cell's proliferation will be inhibited, which will decrease in its activity or even end up with apoptosis. There is a very sensitive regulatory point in T cells to tryptophan content. Under the effect of IDO, tryptophan can be consumed, which makes T cells stagnate in the middle of G1 phase, thus inhibiting the T cell proliferation and their immune response. Once T cells stop proliferating, they may not be stimulated again, which is the immune mechanism of IDO in vivo.

WO 2019/087028 A1 discloses IDO1 inhibitor compounds of Formula (I) and pharmaceutically acceptable salts thereof, their pharmaceutical compositions, their methods of preparation, and methods for their use in the prevention and/or treatment of diseases.

WO 2019/078968 A2 discloses IDO inhibitors and methods for preparing them. The pharmaceutical compositions comprising such IDO inhibitors and methods of using them for treating cancer, infectious diseases, and other disorders are also disclosed.

WO 2017/192840 A1 discloses compounds that modulate or inhibit the enzymatic activity of indoleamine 2,3-dioxygenase (IDO), pharmaceutical compositions containing said compounds and methods of treating proliferative disorders, such as cancer, viral infections and/or inflammatory disorders utilizing the compounds of the disclosure.

CN 105732636 A discloses hetero-aromatic compounds and applications thereof in pharmacy. Specifically, the disclosure provides hetero-aromatic compounds, and steric isomers, geometric isomers, tautomers, racemic bodies, nitrogen oxides, hydrates, solvates, metabolism products, and pharmaceutically acceptable salts or prodrug thereof.

A new type of IDO inhibitor with high activity still waits to be developed in the field, and a novel compound of spiro structure has been found with unexpectedly high IDO inhibitory activity in the invention.

### Contents of the invention

The invention is set out in the appended set of claims. Aspects not falling within the scope of the claims are disclosed and for information only. The invention aims to provide a novel series of compounds of spiro structure as efficient IDO enzyme inhibitors.

Disclosed herein but not covered by the claimed invention is a preparation method of such compounds.

In the first aspect, the present invention provides a compound selected from the group of
*N*-(4-fluorophenyl)-7-(6-fluoroquinolin-4-yl)spiro[3.5]nonane-2-carboxamide,
*N*-(4-chlorophenyl)-7-(6-fluoroquinoline-4-yl)-2-azaspiro [3.5] nonane-2-carboxamide,
*N*-(4-bromophenyl)-7-(6-fluoroquinoline-4-yl)-2-azaspiro [3.5] nonane-2-carboxamide,
*N*-(4-cyanophenyl)-7-(6-fluoroquinoline-4-yl)-2-azaspiro [3.5] nonane-2-carboxamide, and
*N*-(4-fluorophenyl)-7-(6-fluoroquinoline-4-yl)-2-azaspiro [3.5] nonane-2-carboxamide
or their stereoisomers or tautomers, or pharmaceutically-acceptable salts. Disclosed herein are further compounds of Formula (I) or its stereoisomer/tautomer or a pharmaceutically acceptable salt/prodrug not covered by the claimed invention: 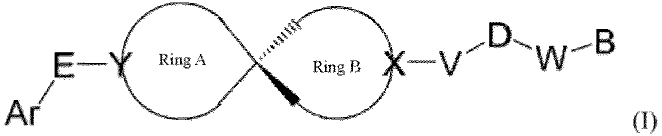 Wherein,
   Ar represents C₆-C₂₀ aryl and C₅-C₂₀ heteroaryl; Ar can be substituted by one or more groups selected from the groups as follows: Halogen, C₁-C₆ alkyl, C₁-C₆ halogenated alkyl, C₁-C₆ alkoxy, hydroxyl, amino, nitro, formyl, -CF₃, -CN, - SF₅, NR^{a}R^{b}, carboxyl, -COR^{a}, -CO₂C₁-C₆ alkyl, - CONR^{a}R^{b}, -SO₂R^{e}, -SO₂NR^{a}R^{b}, -P(O)Me₂ and -P(O)(OMe)₂; wherein, R^{a} and R^{b} are independently hydrogen, substituted/non-substituted C₁-C₁₀ alkyl, substituted/non-substituted C₃-C₁₀ cycloalkyl, substituted/non-substituted C₂-C₁₀ alkenyl, substituted/non-substituted C₆-C₂₀ aryl, or substituted/non-substituted C₃-C₁₄ heteroaryl, substituted/non-substituted C₁-C₁₀ alkynylene -C₆-C₁₀ aryl, substituted/non-substituted C₁-C₁₀ alkynylene-C₂-C₁₀ heteroaryl; R^{a} and R^{b} can form 3-8 membered rings or 4-8 membered heterocyclic rings, the heteroatoms in which may be sulfur, oxygen, NH or NR^{b};
   E is a chemical bond, -O-, -S-, -NR^{a}-, -C (R^{a}) = or -C (R^{a}R^{b})₂-;
   Y is C (R¹), =C, N;
   X is C (R¹), N;
   R¹ is hydrogen, OH, OC₁-C₁₀ alkyl and C₁-C₁₀ alkyl;
   Ring A is connected with Ring B in a spiro structure;
   Ring A and Ring B can be independently a 3-12 membered carbocycle respectively; or
   Ring A and Ring B can be independently a 3-12 membered bicyclic ringrespectively, or
   Ring A and Ring B may be independently a 3-12 membered bridged bicyclic ring respectively, or
   Ring A and Ring B may be a 3-12 membered carbocycle, one or more carbocycle atoms of which may be substituted by one or more O, S, -C(O)-, -C(S)-, NR^{b}, or
   Ring A and Ring B can be a 3-12 membered bicyclic ring, which are non-substituted or are substituted by one or more R^{c}; or
   Ring A and Ring B can be 3-12 membered carbocycle, of which one carbocycle atom can be substituted by a nitrogen atom;
   V is a chemical bond or C₁-C₆ alkylene; V can be substituted by 1 to 3 groups selected from: C₁-C₆ alkyl, OC₁-C₆ alkyl and C₃-C₆ cycloalkyl; or V is NR^{b} or CR^{f}R^{g}; R^{f} and R^{g} can form 3-8 membered rings or 4-8 membered heterocyclic rings, the heteroatoms in which may be sulfur, oxygen, NH or NR^{b};
   R^{f} and R^{g} are independently hydrogen, C₁-C₆ alkyl, C₃-C₆ cycloalkyl, aryl, heteroaryl, C₁-C₆ alkylene aryl, C₁-C₆ alkylene heteroaryl, C₁-C₆ alkylene-C₃-C₆ cycloalkyl respectively; R^{f} can be substituted by one or more substituents selected from C₁-C₆ alkyl, OC₁-C₆ alkyl or C₃-C₆ cycloalkyl;
   D is C (O), C (=NOH), C(S) or S (O)₂;
   W is a chemical bond, -O-, -CR^{a}R^{b}- or -N(R⁵)-;
   R⁵ is hydrogen, C₁-C₆ alkyl, aryl and heteroaryl;
   B represents C₆-C₂₀ aryl and C₅-C₂₀ heteroaryl; B can be substituted by one or more groups selected from: halogen, C₁-C₆ alkyl, C₁-C₆ halogenated alkyl, C₁-C₆ alkoxy, hydroxyl, amino, nitro, formyl, -CF₃, -CN, - SF₅, NR^{a}R^{b}, carboxyl, -COR^{a}, -CO₂C₁-C₆ alkyl, -CONR^{a}R^{b}, -SO₂R^{e}, -S02NR^{a}R^{b}, -P(O)Me₂ and -P(O)(OMe)₂; wherein R^{a} and R^{b} are independently hydrogen, substituted/non-substituted C₁-C₁₀ alkyl, substituted/non- substituted C₃-C₁₀ cycloalkyl, substituted/non-substituted C₂-C₁₀ alkenyl, substituted/non-substituted C₆-C₂₀ aryl, or substituted/non-substituted C₃-C₁₄ heteroaryl respectively; R^{a} and R^{b} can form 3-8 membered rings or 4-8 membered heterocyclic rings, the heteroatoms in which may be sulfur, oxygen, NH or NR^{b};
   R^{e} represents C₁-C₁₀ alkyl, C₃-C₁₀ cycloalkyl, C₆-C₂₀ aryl, or C₃-C₁₄ heteroaryl; R^{e} can be substituted by one or more radical groups selected from: halogen, hydroxyl, amino, nitro, cyano, formyl, carboxyl, alkoxy, -CF₃ and -SF₅.
   Ar may be: 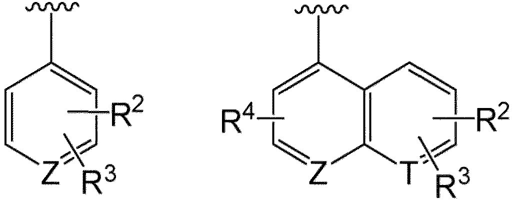 Wherein:
      Z and T are independently CH, CR^{e} or N;
      R², R³ and R⁴ are independently hydrogen, halogen, C₁-C₆ halogenated alkyl, hydroxyl, amino, nitro, formyl, -CF₃, -CN, - SF₅, NR^{a}R^{b}, carboxyl, -COR^{a}, -CO₂C₁-C₆ alkyl, -CONR^{a}R^{b}, -SO₂R^{e}, - SO₂NR^{a}R^{b}, -P(O)Me₂ and -P(O)(OMe)₂. 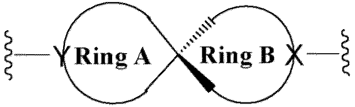 Ring A Ring B of Formula (I) may be selected from: 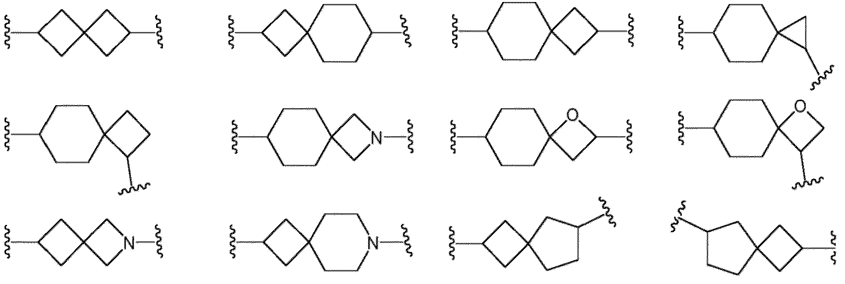 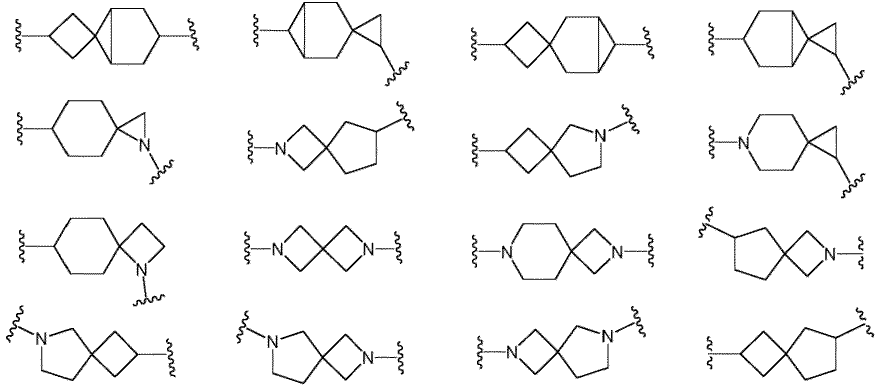 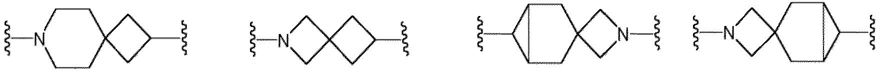
      E in Formula (I) may represent a chemical bond or O.
      Y in Formula (I) may represent CH.
      X in Formula (I) may represent a CH or N.
      V in Formula (I) may represent a chemical bond, -C(C₁-C₆ alkyl)-, -N(R⁵), or -N(CH₂Ar¹)-; Ar¹ represents substituted/non-substituted phenyl; Ar¹ can be substituted by one or more groups selected from: halogen, C₁-C₆ alkyl and C₁-C₆ halogenated alkyl.
      D in Formula (I) may represent -C (O) - or -C(=NOH)-.
      W in Formula (I) may represent a chemical bond or -N (R⁵).
      B in Formula (I) may represent a substituted/non-substituted phenyl; B can be substituted by one or more groups selected from: halogen, C₁-C₆ alkyl, C₁-C₆ halogenated alkyl, C₁-C₆ alkoxy, hydroxyl, -CN, and -SF₅.

The compound of Formula (I) may be as shown Formula (II):

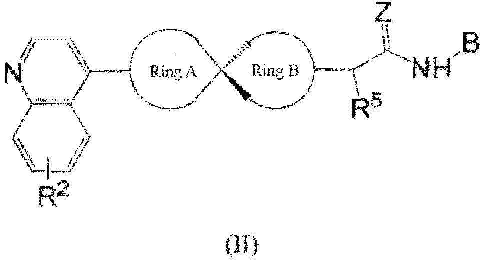

Wherein, R², Ring A, Ring B and B are defined as stated in Formula (I); R⁵ represents hydrogen, C₁-C₆ alkyl, OC₁-C₆ alkyl, C₃-C₆ cycloalkyl and C₁-C₆ alkylene-aryl respectively; Z represents O or NOH.

The compound of Formula (I) may be as shown Formula (III):

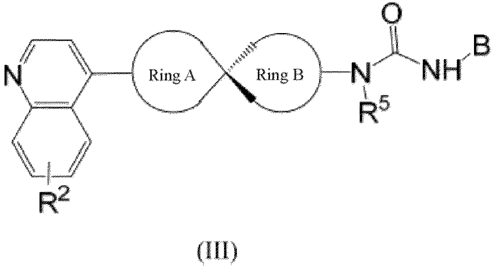

Wherein, R², Ring A, Ring B and B are defined as described Formula (I); R⁵ represents hydrogen, C₁-C₆ alkyl, C₃-C₆ cycloalkyl, and C₁-C₆ alkynylene aryl.

The compound of Formula (I) may be as shown as Formula (IV):

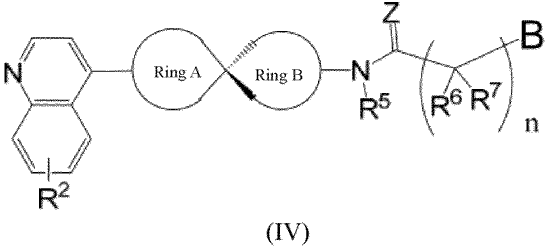

Wherein, R², Ring A, Ring B and B are defined as for Formula (I); R⁵, R⁶ and R⁷ represent hydrogen, C₁-C₆ alkyl, OC₁-C₆ alkyl, C₃-C₆ cycloalkyl and C₁-C₆ alkylene-aryl respectively; R⁶ and R⁷ can form 3-8 membered rings or 4-8 membered heterocyclic rings, the heteroatoms in which may be sulfur, oxygen, NH or NR^{b}; n represents an integer of 1 to 6; Z represents O or NOH.

The compound of Formula (I) may be as shown as Formula (V):

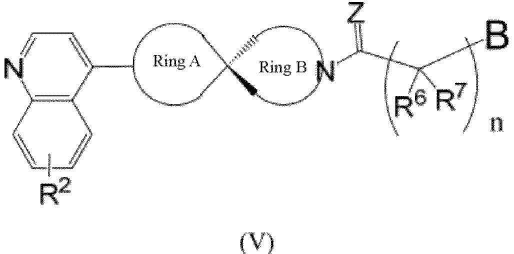

Wherein, R², Ring A, Ring B and B are defined as for Formula (I); R⁵, R⁶ and R⁷ represent hydrogen, C₁-C₆ alkyl, OC₁-C₆ alkyl, C₃-C₆ cycloalkyl and C₁-C₆ alkylene-aryl respectively; R⁶ and R⁷ can form 3-8 membered rings or 4-8 membered heterocyclic rings, the heteroatoms in which may be sulfur, oxygen, NH or NR^{b}; n represents an integer to 1 to 6; Z represents O or NOH.

The compound of Formula (I) may be shown as Formula (VI),

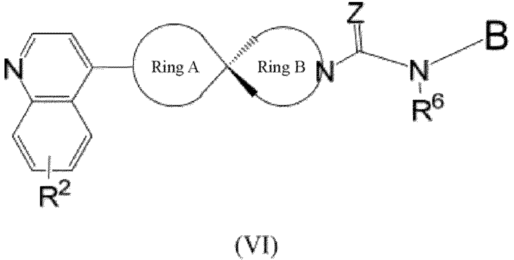

Wherein, R², Ring A, Ring B and B are defined as for Formula (I); R⁶ represents hydrogen, C₁-C₆ alkyl, OC₁-C₆ alkyl, C₃-C₆ cycloalkyl and C₁-C₆ alkylene-aryl respectively; Z represents O or NOH.

The compound of Formula (I) may be shown as Formula (VII):

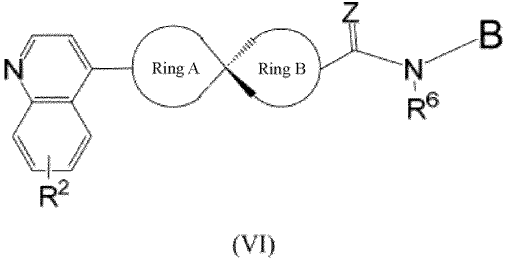

Wherein, R², Ring A, Ring B and B are defined as stated in Claim 1; R⁶ represents hydrogen, C₁-C₆ alkyl, OC₁-C₆ alkyl, C₃-C₆ cycloalkyl and C₁-C₆ alkylene-aryl respectively; Z represents O or NOH.

The compound of Formula (I) may be shown as Formula (VIII):

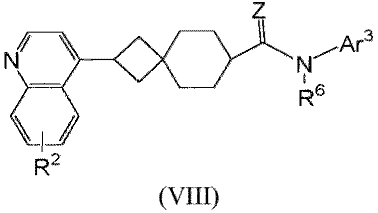

Wherein, R² represents halogen, and R⁶ represents hydrogen, C₁-C₆ alkyl, C₃-C₆ cycloalkyl and C₁-C₆ alkylene aryl; Z represents O or NOH; Ar³ represents substituted/non-substituted phenyl, and the substituent can be selected from halogen, C₁-C₆ alkyl, C₁-C₆ halogenated alkyl, C₁-C₆ alkoxy, hydroxyl, amino, nitro, -CF₃, -CN, -SF₅, NR^{a}R^{b}, carboxyl, -COR^{a}, -CO₂C₁-C₆ alkyl, -CONR^{a}R^{b}, - SO₂R^{e}, -S02NR^{a}R^{b}, -P(O)Me₂, and -P(O)(Me)₂, wherein, R^{a} and R^{b} are defined as for Formula (I).

The compound of Formula (I) may be shown as Formula (IX):

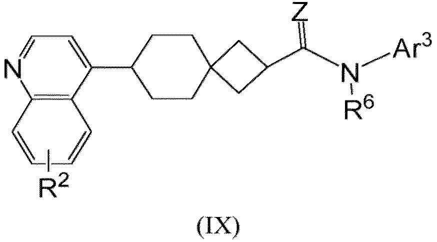

Wherein, R² represents halogen, and R⁶ represents hydrogen, C₁-C₆ alkyl, C₃-C₆ cycloalkyl and C₁-C₆ alkylene-aryl; Z represents O or NOH; Ar³ represents substituted/non-substituted phenyl, and the substituent can be selected from halogen, C₁-C₆ alkyl, C₁-C₆ halogenated alkyl, C₁-C₆ alkoxy, hydroxyl, amino, nitro, -CF₃, -CN, -SF₅, NR^{a}R^{b}, carboxyl, -COR^{a}, -CO₂C₁-C₆ alkyl, -CONR^{a}R^{b}, - SO₂R^{e}, -S02NR^{a}R^{b}, -P(O)Me₂, and -P(O)(Me)₂, wherein, R^{a} and R^{b} are defined as for Formula (I).

In a preferred embodiment, the stereoisomer is a cis-trans isomer.

In another preferred embodiment, the compound is a racemate.

In another preferred embodiment, the stereoisomer is an enantiomer.

It is disclosed herein but not covered by the claimed invention that any hydrogen in the compound may be substituted by deuterium.

In another preferred embodiment, the pharmaceutically acceptable salt is selected from the groups as follows: hydrochloride, hydrobromide, sulfate, phosphate, mesylate, trifluoromethylsulfonate, benzenesulfonate, p-toluenesulfonate (tosylate), 1-Naphthalenesulfonate, 2-naphthalenesulfonate, acetate, trifluoroacetate, malate, tartrate, citrate, lactate, oxalate, succinate, fumarate, maleate, benzoate, salicylate, phenylacetate and mandelate.

The compound in Formula (I) can be obtained with the following preparation methods, including the steps as follows:

### Method 1

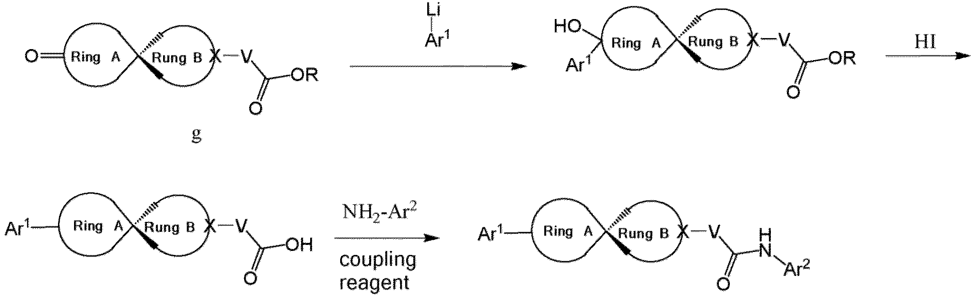

Wherein, R represents C₁-C₆ alkyl and C₁-C₆ halogenated alkyl; the definition of Ar² is the same as that of B; the definition of Ar¹ is the same as that of Ar, and the other groups or atoms are defined the way above mentioned.

### Method 2

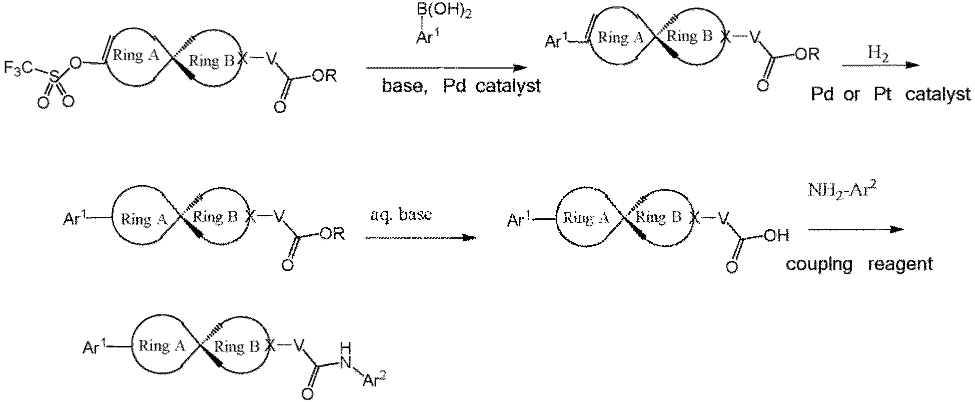

herein, R represents C₁-C₆ alkyl and C₁-C₆ halogenated alkyl; the definition of Ar² is the same as that of B; the definition of Ar¹ is the same as that of Ar, and the other radical groups or atoms are defined the way above mentioned.

### Method 3

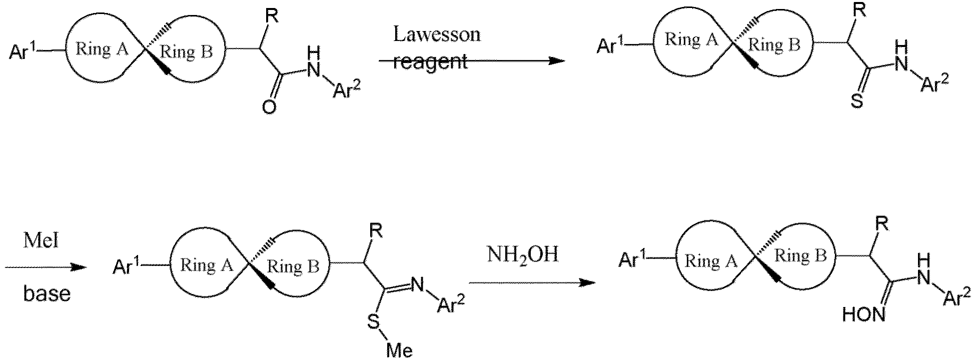

Wherein, the definition of -CHR- is the same as that of V; the definition of Ar² is the same as that of B; the definition of Ar¹ is the same as that of Ar, and the other groups or atoms are defined the way as above mentioned.

### Method 4

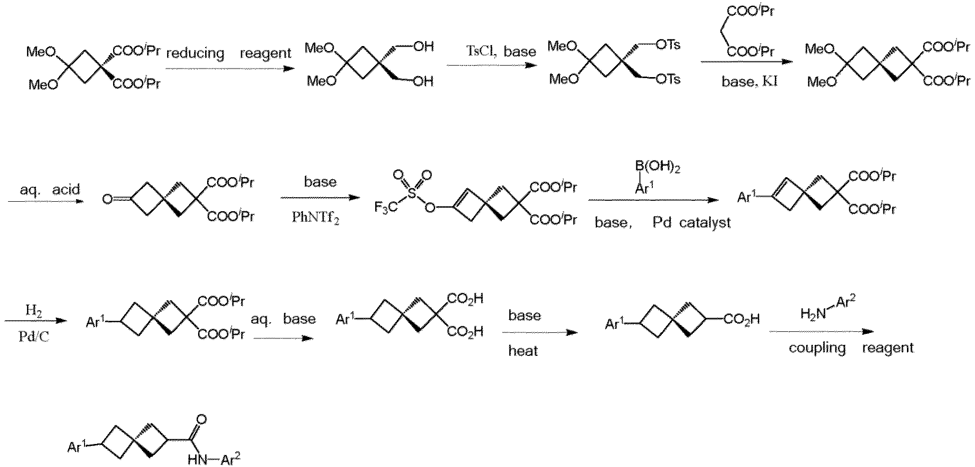

Wherein, the definition of Ar² is the same as that of B; the definition of Ar¹ is the same as that of Ar.

### Method 5

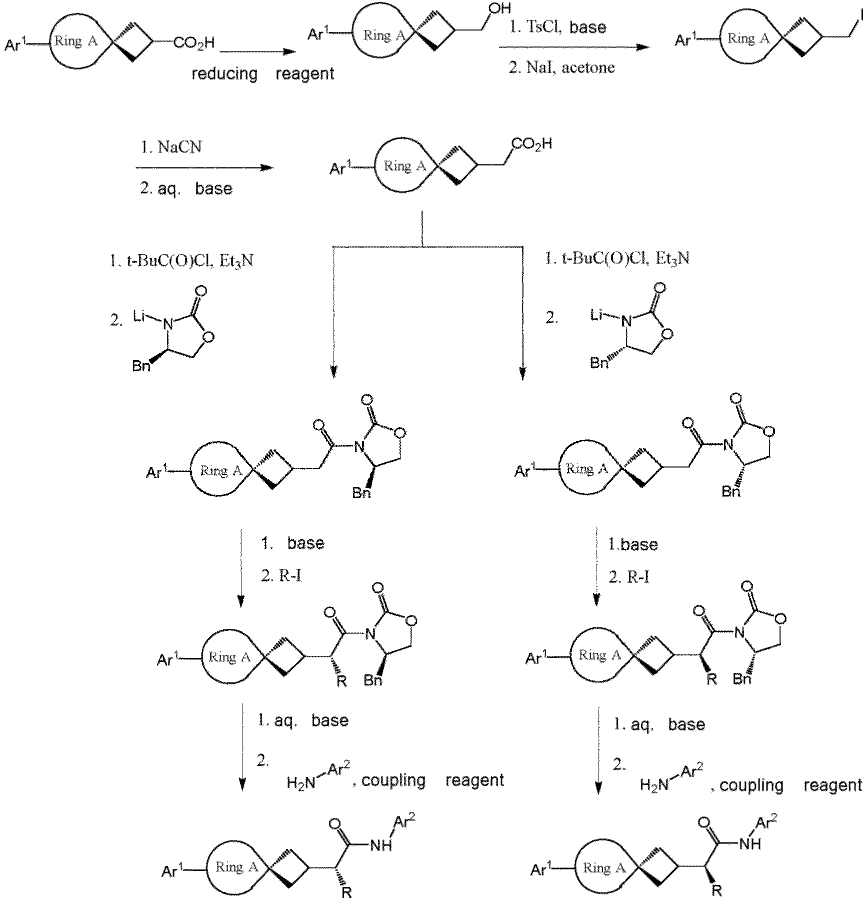

Wherein, the definition of CHR is the same as that of V; the definition of Ar² is the same as that of B; the definition of Ar¹ is the same as that of Ar, and the other groups or atoms are defined the way above mentioned.

### Method 6

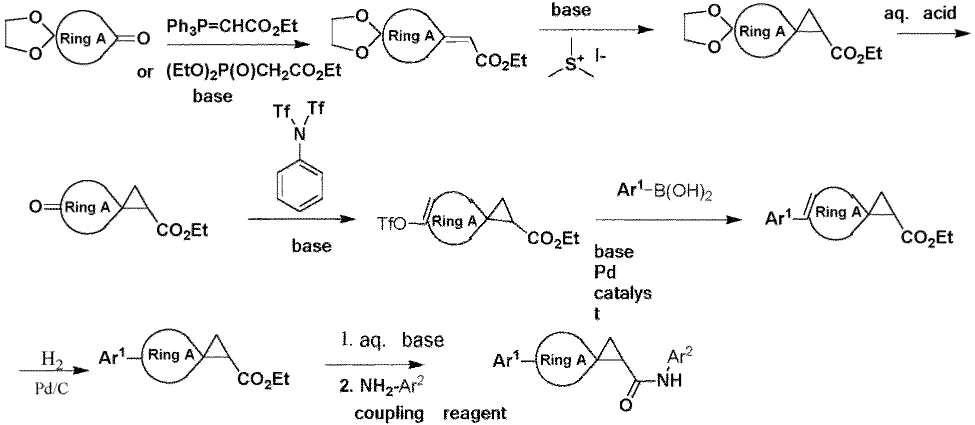

Wherein, the definition of Ar² is the same as that of B; the definition of Ar¹ is the same as that of Ar, and the other groups or atoms are defined the way above mentioned.

### Method 7

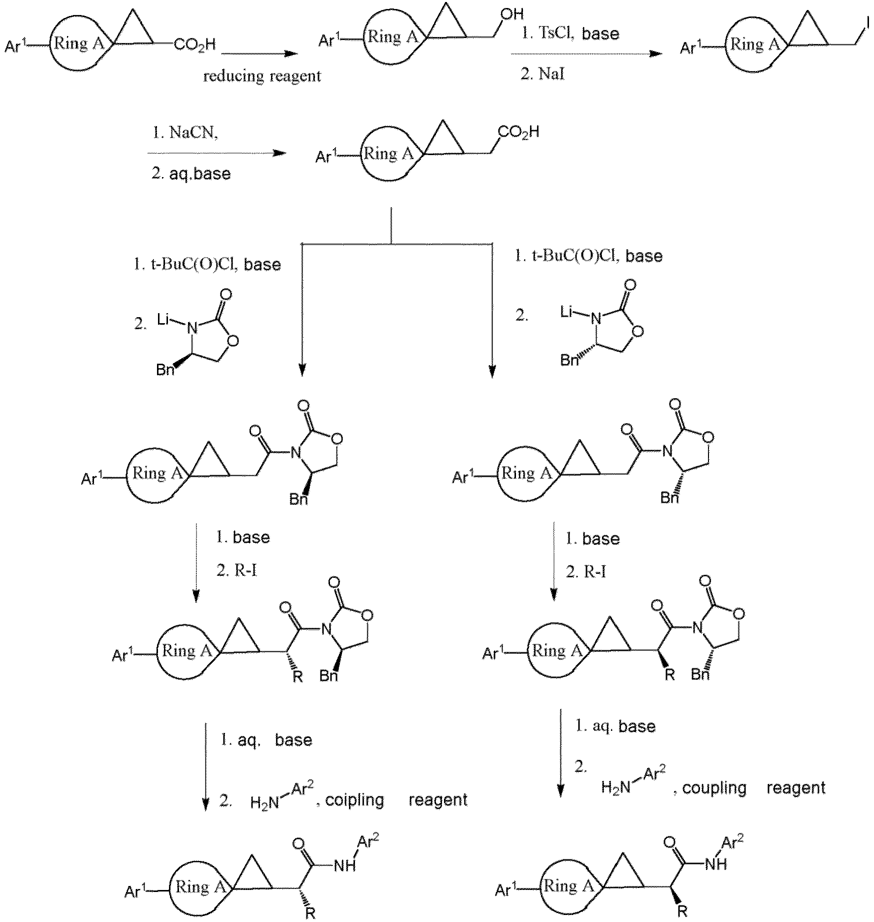

Wherein, the definition of CHR is the same as that of V; the definition of Ar² is the same as that of B; the definition of Ar¹ is the same as that of Ar, and the other groups or atoms are defined the way above mentioned.

### Method 8

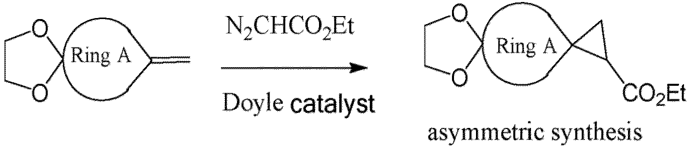

### Method 9

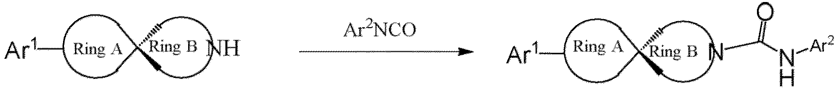

Wherein, the definition of CHR is the same as that of V; the definition of Ar² is the same as that of B; the definition of Ar¹ is the same as that of Ar, and the other groups or atoms are defined the way as above mentioned.

Of the above methods,

The base can be selected from the groups as follows: alkali hydroxides, alkali-earth hydroxides, alkali hydride, HMDS alkali metal salt, pyridine, triethylamine, etc.

The acids can be selected from the groups as follows: Hydrochloride, sulfuric acid, etc.

The coupling reagents can be selected from the groups as follows: HATU, etc.

The palladium catalyst can be selected from the groups as follows: tetrakispalladium, etc.

The catalyst can be selected from the groups as follows: palladium-carbon catalyst, etc.

The reductant can be selected from the groups as follows: LiAlH₄, etc.

On the other hand, the invention provides a compound or its stereoisomer/tautomer mentioned in the first aspect or a pharmaceutically acceptable salt, which are used in:
(i) Preparation of indoleamine-2, 3-dioxygenase inhibitor;
(ii) Preparation of drugs for the prevention and/or treatment of indoleamine-2, 3-dioxygenase mediated diseases; or
(iii) Preparation of anti-inflammatory drugs. Prodrugs of such compound are disclosed, but not covered by the claimed invention.

The invention provides the compounds of the first aspect, or their stereoisomers or tautomers, or pharmaceutically-acceptable salts for use as an antitumor drug. The invention further provides the compounds of the first aspect, or their stereoisomers or tautomers, or pharmaceutically-acceptable salts for use in the prevention and/or treatment of indoleamine-2,3-dioxygenase mediated diseases.

In another preferred embodiment, the indoleamine-2, 3-dioxygenase mediated diseases are those with pathological characteristics of IDO mediated tryptophan metabolism pathway.

The indoleamine-2, 3-dioxygenase mediated diseases are selected from cancer, neurodegenerative disease, HIV infections, eye disease, psychological disorder, depression, anxiety disorder, Alzheimer's disease and/or autoimmune diseases.

In another preferred embodiment, the cancer includes but not limited to: colon cancer, breast cancer, gastric cancer, lung cancer, colorectal cancer, pancreatic cancer, ovarian cancer, prostate cancer, kidney cancer, liver cancer, brain cancer, melanoma, multiple myeloma, chronic myeloid leukemia, hematologic tumor, lymphoma, including metastases in other tissues or organs far away from the primary tumor lesions.

The invention also provides a pharmaceutical composition comprising:
A compound or its stereoisomer/tautomer mentioned in the first aspect of the invention or a pharmaceutically acceptable salt; and pharmaceutically acceptable carriers.

The pharmaceutical composition also contains other antitumor drugs.

In another preferred embodiment, the other antitumor drugs are selected from the groups as follows: PD-1 antibody, PD-L1 antibody, CTLA-4 antibody and other antitumor chemotherapy drugs and targeted drugs.

In another preferred embodiment, the other antitumor drugs include, but are not limited to, immunotherapeutic drugs against cancer: PD-1 antibody, CTLA-4 antibody, PD-L1 antibody, PD-L2 antibody, any other chemotherapy drug or targeted therapeutic drug, such as HDAC inhibitor, inhibitor of arginine metabolic enzyme, STING activator and EP4 antagonist.

Disclosed herein but not covered by the claimed invention is a prevention and/or treatment of indoleamine-2, 3-dioxygenase mediated diseases, including the steps giving a patient such a compound of Formula (I) or of the invention as described above or its stereoisomer or tautomer, or its pharmaceutically acceptable salt or prodrug or pharmaceutical composition above.

The indoleamine-2, 3-dioxygenase mediated diseases may refer to cancers, and the methods further consist of steps of applying additional anticancer agents (also known as antitumor drugs, as described above) to a patient.

The compound of the invention has such pharmacological activities as anti-tumor, treatment of neurodegenerative diseases (Alzheimer's disease), and anti-inflammatory.

It is noteworthy that, within the scope of the invention, the technical features as mentioned above and described in detail below (factual examples) can be combined with each other to form a new or preferred technical solution, which will not be repeated here due to limited length.

### Specific implementation proposals

With the extensive and in-depth research by the present inventors, a new compound of the structure with spiro features was accidentally developed, which can be used as an efficient IDO enzyme inhibitor to prevent and/or treat indoleamine-2,3-dioxygenase mediated diseases, and used as anti-inflammatory drugs. On such basis, the invention was completed.

### Definition

The term "C₁-C₁₀ alkyl" refers to monovalent saturated aliphatic hydrocarbyl with 1-10 carbon atoms, including straight-chain and branched-chain hydrocarbyl, such as methyl (CH₃-), ethyl (CH₃CH₂-), n-propyl (CH₃CH₂CH₂-), isopropyl ((CH₃)₂CH-), n-butyl (CH₃CH₂CH₂CH₂-), isobutyl ((CH₃)₂CHCH₂-), sec-butyl ((CH₃)(CH₃CH₂)CH-), tert-butyl ((CH₃)₃C-), and n-amyl (CH₃CH₂CH₂CH₂CH₂-), and neopentyl ((CH₃)₃CCH₂-). In the invention, the term includes substituted/non-substituted alkyl.

As used herein, the term " substituted/non-substituted " means that the groups can be non-substituted or that H in the radical group is substituted by one or more (preferably 1-6, more preferably 1-3) substituents.

As used herein, the term " substituted/non-substituted " means that the groups have one or more (preferably 1-6, more preferably 1-3) substituents selected from the groups as follows: halogen, hydroxyl, -NH₂, nitro, -CN, C₁-C₆ alkyl, C₁-C₆ halogenated alkyl, C₁-C₆ alkoxy, C₃-C₆ cycloalkyl, C₂-C₆ alkenyl, C₂-C₆ alkynyl, phenyl, benzyl, C₁-C₆ alkylS(O)₂-, (C₀-C₆ alkyl)₂NS(O)₂-, C₁-C₆ alkyl-C(O)-, C₃-C₆ cycloalkyl-C(O)-, C₀-C₆ alkyl-OC(O)-, (C₀-C₆ alkyl)₂NC(O)-, C₀-C₆ alkyl-C(O)NH-, and (C₀-C₆ alkyl)₂NC(O)NH-.

As used herein, the term "C₃-C₁₂ cycloalkyl" refers to a cyclic, substituted/non-substituted cycloalkyl with 3-12 carbon atoms, such as -CH₂-cyclopropane and -CH₂-cyclobutane.

As used herein, the term "alkoxy" refers to -O-alkyl, of which the alkyl may be saturated/unsaturated, may be branched-chain, straight-chain, or cyclic. Preferably, the alkoxy has 1-10 carbon atoms, namely C₁-C₁₀ alkoxys, preferably 1-6 carbon atoms. Representative examples include, but are not limited to: methoxyl, ethoxyl and propoxyl.

As used herein, the term "C₆-C₂₀ aryl" refers to a monocyclic (e.g. phenyl) or condensed ring (e.g. naphthyl or anthracyl) with 6-20 (preferably 6-14) carbon atoms, and the condensed ring may be nonaromatic (e.g. 2-benzoxazolinone, 2*H*-1, 4-benzoxazolinone-3(4*H*)-keone-7-yl, etc.) if the attachment point is on the aromatic carbon. The preferred aryl consists of phenyl and naphthyl. The term includes substituted/non-substituted forms, of which substituents are defined as above.

As used herein, the term "C₂-C₁₀ alkenyl" refers to an alkenyl with 2-10 (e.g. 2-6 or 2-4) carbon atoms and at least 1 (e.g. 1-2) unsaturated olefinic bonds (>C = C<). Such radical groups consist of ethenyl, allyl, and but-3-enyl.

As used herein, the term "C₃-C₁₀ cycloalkyl" refers to a cyclic alkyl with 3-10 carbon atoms and single/multiple rings (including fused, bridged and spiro systems). In a fused ring system, one or more rings can be cycloalkyl, heterocyclic, aryl or heteroaryl, provided the connecting sites are cycloalkyl rings. Examples of suitable cycloalkyls include: Adamantyl, cyclopropyl, cyclobutyl, cyclopentyl, and cyclooctyl.

As used herein, the term "halogenated"/"halogen" refers to fluorine, chlorine, bromine and iodine.

As used herein, the term "heteroaryl" refers to aromatic radical groups with 1-10 carbon atoms and 1-4 heteroatoms selected from oxygen, nitrogen and sulfur in the rings. As for the terms of trees indicating carbon atoms, for example, "C₃-C₂₀ heteroaryl" denotes aromatic groups with 3-20 carbon atoms and 1-4 heteroatoms selected from oxygen, nitrogen and sulfur. Others are similar. Such heteroaryl can be monocyclic (e.g., pyridyl or furyl) or condensed ring (such as indolizinyl or benzothiophene), of which the condensed rings can be non aromatic and/or contain a heteroatom, provided the connecting sites are atoms of the aromatic heteroaryl. In one example, ring nitrogen and/or sulfur of heteroaryl are selectively oxidized to N-oxide (N-O), sulfinyl or sulfonyl. Preferably, heteroaryls consists of pyridyl, pyrryl, indolyl, thienyl and furyl. The term includes substituted/non-substituted heteroaryl.

As used herein, the term "substituted heteroaryl" refers to a heteroaryl substituted by 1-5, preferably 1-3, more preferably 1-2 substituents selected from the substituent defined similarly with substituted aryl.

As used herein, the term "heterocyclic ring"/"heterocyclic"/"heterocyclic alkyl"/"heterocyclyl" refers to a saturated, partially saturated or unsaturated radical group (but not aromatic), with single rings or condensed rings (including a bridged ring system and a spiro system) in which there are 1-10 carbon atoms and 1-4 (e.g. 3) heteroatoms selected from nitrogen, sulfur or oxygen. In a condensed ring system, one or more rings can be cycloalkyl, aryl or heteroaryl, only if the connecting sites pass through the nonaromatic rings. In one example, nitrogen and/or sulfur atoms of a heterocyclyl radical group are selectively oxidized to provide N-oxide, sulfinyl and sulfonyl.

As used herein, the term "substituted heterocyclic"/"substituted heterocyclic alkyl"/"substituted heterocyclyl" refers to a heterocyclic group substituted by 1-5 (e.g. 1-3) substituents which are the same as the substituent defined by the substituted cycloalkyl.

As used herein, the term "stereoisomer" refers to compounds with different chirality in stereocenters. Stereoisomers include enantiomers and diastereomers.

As used herein, the term "tautomer" refers to alternative forms of compounds with different proton locations, such as tautomer of enol ketone and imine enamine, or tautomeric forms of heteroaryl which contains ring atoms connected to the -NH- and =N- of the ring, such as pyrazol, imidazole, benzimidazole, triazole and tetrazole.

"Prodrug" refers to any derivative of the example compound, which can directly or indirectly provide the example compound, its active metabolite or residue when being applied to a subject. Particularly preferred derivatives and prodrugs are those that improve the bioavailability of the example compound (e.g. the compound administered orally tends to be absorbed in the blood more easily) or the delivery of the matrix compound to a biological compartment (such as brain or lymphocytic system) as per the matrix type when being applied to a subject. Prodrugs include ester forms in the compounds of the invention.

### Compounds in the invention

As used herein, the term "compounds in the invention" refers to compounds according to claim 1, their racemes, stereoisomers or tautomers, or pharmaceutically acceptable salts.

The invention relates to: racemic mixtures of such compounds, mixtures enriched in any enantiomer, and separated enantiomer. The scope of the invention shall be understood the way that the racemic mixture refers to 50% of two R and S enantiomers: 50% of the mixture. The separated enantiomers shall be understood as pure enantiomers (i.e. 100%) or mixtures with highly enriched enantiomers (purity ≥98%, ≥95%, ≥93% ^90%, ≥88%, ≥85% and ≥80%).

Where the compounds contain stereoisomers as mentioned in the invention, the invention shall include all the stereoisomers of such compounds.

Where the compounds contain tautomers as mentioned in the invention, the invention shall include all the tautomers of such compounds.

Also disclosed but not covered by the claimed invention are deuterated compounds generated from the replacement of any one/more hydrogen atoms in the compound with its/their stable isotope deuterium.

### Pharmaceutical composition

The invention also provides a pharmaceutical composition, which contains active ingredients at a safe and effective dosage, and pharmaceutically acceptable carriers.

The "active ingredient" in the invention refers to the compounds of the invention or their stereoisomers or tautomers, or pharmaceutically acceptable salts.

The "active ingredient" and the pharmaceutical composition in the invention can be used as IDO inhibitors. In another preferred embodiment, it is used to prepare drugs for the prevention and/or treatment of tumors. In another preferred embodiment, it is used to prepare drugs for the prevention and/or treatment of IDO mediated diseases.

"Safe and effective dosage" means: The dosage of active ingredient is sufficient to improve the condition without serious side effects significantly. Generally, the pharmaceutical composition contains 1-2,000mg of the active ingredient/agent; preferably, it contains 10-200mg of the active ingredient/agent. More preferably, "one dosage" is contained in a tablet.

"Pharmaceutically acceptable carrier" means: One or more compatible solid/liquid fillers or gel substances, which are suitable for human use, and shall be of sufficient purity and low toxicity. "Compatibility" here refers to the fact that each component of the composition can be blended with and among the active ingredients in the invention without significant reduction of the active ingredient's efficacy.

The compound of the invention can be administered as a single active agent or in combination with one or more other agents for cancer treatment. The combination of the compound of the invention with the known therapeutic agents and anticancer agents is also effective; the combination of the known compounds with other anticancer agents or chemotherapy agents is within the scope of the preferred embodiments. Examples of such agents can be seen in Cancer Principles and Practice of Oncology, V T Devita & S Hellman (Editor), Edition VI (February 15, 2001), Lippincott Williams & Wilkins. Based on the special properties of drugs and cancers involved, an ordinary technician in the field can identify the effective drug combinations. Such anticancer agents include (but are not limited to) the ones as follows: Estrogen receptor modulators, androgen receptor modulators, retinol receptor modulators, cytotoxic/cell growth inhibitors, anti-proliferation agents, isopentenyl protein transferase inhibitors, histone deacetylase (HDAC) inhibitors, HMG-CoA reductase inhibitors and other angiogenesis inhibitors, inhibitors of cell proliferation and survival signal, apoptosis inducers, interference cell cycle checkpoint , CTLA4 antibody, PD-1 antibody, PD-L1 antibody, etc. The compounds of the invention are also effective when administered in combination with radiotherapy.

In general, the compounds of the invention will be administered in a therapeutically effective dosage and any acceptable mode via any medicament of a similar effect. The actual dosages of the compounds (i.e. active ingredients) in the preferred embodiments are determined based on numerous factors, such as the severity of diseases to be treated, age and relative health of a patient, efficacy of the compounds used, and route & form of application. The drug may be administered for times a day (once or twice preferably a day). All of such factors are taken into account by the attending physician.

For the purpose of the preferred embodiment, the therapeutically effective dosage can be a daily total dosage generally, for example, from 0.001-1,000mg/kg weight for one time or times (preferably 1.0-30mg/kg weight per day for a patient). Dosage unit composition can include its dosage factors to form a daily dosage. The dosage forms are chosen depending on various factors, such as administration mode and bioavailability of drug substances. In general, the compounds of the invention can be administered as a pharmaceutical composition through any of the routes as follows: Oral, systemic (e.g. transdermal, intranasal or suppository), or parenteral (e.g. intramuscular, intravenous or subcutaneous). The preferred method of administration is oral, whose convenient daily dosage can be adjusted as per the bitterness. The composition may be made in the forms of tablet, pill, capsule, semi-solid, powder, sustained-release preparation, solution, suspension, elixir, aerosol or any other appropriate composition. Another preferred administration mode of compounds of the invention is inhalation, which is an effective mode to deliver therapeutic agents directly to the respiratory tract (refer to U.S. Patent No. 5607915 for example).

Pharmaceutically acceptable carriers or excipients include: Treatment agents, drug delivery modifiers and accelerators, such as calcium phosphate, magnesium stearate, talc, monosaccharide, disaccharide, starch, gelatin, cellulose, sodium methylcellulose, carboxymethyl cellulose, glucose, hydroxypropyl-B-cyclodextrin, polyvinylpyrrolidone, low-melting-point wax, ion exchange resin, and any combination of two or more of them. Liquid and semi-solid excipients can be selected from glycerol, propylene glycol, water, ethanol and various oils (including petroleum, animal oil, vegetable oil or synthetic oils, such as peanut oil, soybean oil, mineral oil and sesame oil). The preferred liquid carriers (in particular those for injectable solutions) include water, brine, glucose aqueous solution and ethylene glycol. Other pharmaceutically acceptable excipients are described in Remington's Pharmaceutical Sciences, Mack pub. Co., New Jersey (1991).

As used herein, the term "pharmaceutically acceptable salt" refers to a non-toxic acid or alkaline-earth metal salt of a compound of the invention. Such salts can be prepared in situ while the final separation and purification of compounds of the invention, or via the reaction among proper organic/inorganic acids, alkalis, alkali/acid or functional groups. Representative salts include, but are not limited to: Acetate, adipate, alginate, citrate, aspartate, benzoate, benzene sulfonate, disulfate, butyrate, camphorate, camphorsulfonate, diglucosate, cypionate, lauryl sulfate, esilate, glucose heptanate, glycerophosphate, hemisulphate, enanthate, hexanoate, fumarate, hydrochloride, hydrobromate, hydroiodate, 2-hydroxyethyl sulfonate, lactate, maleate, mesylate, nicotinate, 2-naphthyl sulfonate, oxalate, dihydroxynaphthalate, pectinate, thiocyanate, 3-phenylpropionate, picrate, pivalate, propionate, succinate, sulfate, tartrate, thiocyanate, p-toluenesulfonate and undecanoate. In addition, the basic groups containing nitrogen can be quaternary-ammonium salted with the agents as follows: Alkyl halides such as chlorides, bromides and iodides of methyl, ethyl, propyl and butyl groups; dialkyl sulfates such as dimethyl, diethyl, dibutyl and diamyl sulfate; long-chain halides such as chlorides, bromides and iodides of decyl, lauryl, myristyl and alkyl; aromatic alkyl halides such as benzyl and phenylethyl bromide. Water soluble, oil soluble or dispersible products are obtained. Examples of acids that can be used to form pharmaceutically acceptable acid-addition salts include inorganic acids of hydrochloride, sulfuric acid, phosphoric acid, etc. as well as the organic acids of oxalic acid, maleic acid, methanesulfonic acid, succinic acid, citric acid, etc. Alkali-addition salts can be prepared in situ while final separation and purification of compounds of the invention, or via the reaction of carboxylic acid portion with proper alkali (such as pharmaceutically acceptable hydroxides of metal cations, carbonate or bicarbonate), ammonia, organic primary, secondary or tertiary amines, respectively. Pharmaceutically acceptable salts include, but are not limited to, alkali metal and alkaline-earth metal based cations, such as sodium, lithium, potassium, calcium, magnesium, aluminum salts, as well as non-toxic ammonium, quaternary, and amine cations, including, but not limited to: Ammonium, tetramethyl-ammonium, tetraethylammonium, methylamine, dimethylamine, trimethylamine, triethylamine, ethylamine, etc. Other representative organic amines used to produce alkali-addition salts include diethylamine, ethylenediamine, ethanolamine, diethanolamine, piperazine, etc.

As used herein, the term "pharmaceutically acceptable prodrug" refers to the prodrug of the compound in the preferred embodiment, which converts rapidly in vivo to the matrix compound as shown in the above Formula, such as hydrolysis in blood. Pro-drugs as Novel Delivery Systems (Vol. 14 of aA.C.S. 15 Symposium Series, edited by Edward B. Roche) by T. Higuchi and V. Stella and Bioreversible Carriers in Drug Design (1987) by APA and Pergamon provide a complete discussion.

The invention is advantageous in:
(1) A compound according to claim 1 with novel structure is provided;
(2) The compound in the invention can be used as an effective IDO enzyme inhibitor;
(3) The synthesis conditions are mild and easy to operate with a high yield, easy derivatization, which is suitable for industrial mass production;
(4) The compound has the pharmacological activities such as prevention/treatment of tumor, neurodegenerative disease (Alzheimer's disease) and inflammatory symptom.

The invention will be further construed in combination with specific examples. It is noteworthy that such examples are intended only to construe the invention rather than to limit its scope. The experimental methods without specific conditions in the following examples are usually adopted as per conventional conditions (for example, Sambrook et al., molecular cloning): Conditions mentioned in the Laboratory Manual (New York: Cold Spring Harbor Laboratory Press, 1989), or those recommended by the manufacturers. Percentages and numbers shall be counted by weight unless otherwise stated.

All the professional and scientific terms used in this paper share the same meaning as those familiar to the skilled in the field unless otherwise defined. In addition, any method and material similar to the content described in the paper can be applied to the methods in the disclosure. The preferable implementation methods and materials described in this paper are only for demonstration.

### Example 1 (comparative)

### (±)-N-(4-chlorophenyl)-6-(quinolin-4-yl)spiro[3.3]heptane-2-carboxamide

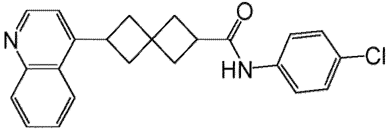

### Step 1: (3,3-dimethoxycyclobutane-1,1-diyl)dimethanol

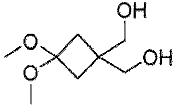

Lithium aluminum hydride (28.81 g, 759.7 mmol) was added into a three-necked flask containing tetrahydrofuran (1,500 mL) in batches. Tetrahydrofuran solution (500 mL) of diisopropyl 3,3-dimethoxycyclobutane-1,1-dicarboxylate (99.57 g, 345.3 mmol) was added slowly. After stirring at room temperature for 12h, TLC showed that the reaction had was completed. Saturated potassium sodium tartrate solution (27 mL) was added to quench the reaction in ice bath, and the mixture was stirred at room temperature for 1h. The mixture was filtered and the filter cake was washed with dichloromethane/methanol (10:1, 200 mL x 5), and the combined organic phase was concentrated to obtain colorless oily product (59.8 g, yield: 95%).

¹HNMR (400 MHz, CDCl₃): δ 1.94 (s, 4H), 3.12 (s, 6H), 3.70 (s, 4H).

### Step 2: (3,3-dimethoxycyclobutane-1,1-diyl)bis(methylene) bis(4-methylbenzenesulfonate)

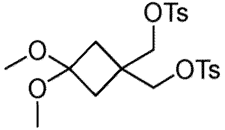

The product of Step 1 (56.7 g, 321.8 mmol) was dissolved in pyridine (500 mL), cooled in ice bath and treated with p-toluenesulfonyl chloride (153.4 g, 804.7 mmol) in batches. After stirring for 12 h at room temperature, TLC showed that the reaction was completed. The reaction solution was filtered and then poured slowly into water (1000 mL). The solid was collected by filtration to obtain the product as a white solid (137.3 g, yield: 88%).

¹H NMR (400 MHz, CDCl₃): δ 1.92 (s, 4H), 2.45 (s, 6H), 3.01 (s, 6H), 3.99 (s, 4H), 7.36 (d, 4H), 7.74 (d, 4H).

### Step 3: diisopropyl 6,6-dimethoxyspiro[3.3]heptane-2,2-dicarboxylate

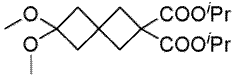

*N,N-*dimethylformamide (300 mL) was added into a three-necked flask, followed by sodium hydride (9.63g, 240.8mmol) in batches. Nitrogen was changed for three times. Diisopropyl malonate (41.61g, 221.1mmol) was added dropwise into the solution and stirred at room temperature for 1h. A solution of the product of Step 2 (53.03 g, 109.4 mmol) and KI (1.82 g, 10.94 mmol) in *N, N-*dimethylformamide (200 mL) was added into the reaction system, and stirred at 140 °C for 12 h. TLC showed that the reaction was completed. The cooled reaction solution was poured into water (1,000 mL), extracted and concentrated with petroleum ether (500 mL x 3) and distilled under reduced pressure (125-127 °C/2 mmHg) to obtain a light-yellow oily product (16.9 g, yield: 47%).

¹H NMR (400 MHz, CDCl₃): δ 1.22 (d, 12H), 2.21 (s, 4H), 2.58 (s, 4H), 3.12 (s, 6H), 5.04 (sep, 2H).

### Step 4: diisopropyl 6-oxospiro[3.3]heptane-2,2-dicarboxylate

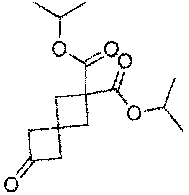

The product of Step 3 (1.00 g, 3.04 mmol) and hydrochloric acid (14 mL, 3M) were placed in a round-bottom flask and stirred at room temperature for 6 h. After the reaction had been completed, white solid were generated which was collected by filtration to give the title product (800 mg, yield: 93%).

¹H NMR (400 MHz, CDCl₃): δ 5.07 (sep, 2H), 3.14 (s, 4H), 2.78 (s, 4H), 1.24 (d, 12H).

### Step 5: diisopropyl 6-(((trifluoromethyl)sulfonyl)oxy)spiro[3.3]hept-5-ene-2,2-dicarboxylate

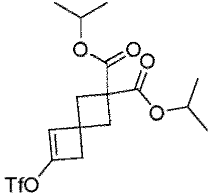

The product (700 mg, 2.48 mmol) of Step 4 and dried tetrahydrofuran were added into a three-necked flask. Nitrogen was filled for three times. The solution was cooled to -78°C in dry-ice acetone bath. NaHMDS tetrahydrofuran solution (1.49 mL, 2M, 2.98 mmol) was slowly added into the solution and stirred under the protection of nitrogen at -78 °C . *N-*phenylbis(trifluoromethanesulfonimide) solution (1.06 g, in 15 mL of tetrahydrofuran) was slowly added into the solution and stirred at -78°C for 0.5 h under N₂. Saturated ammonium chloride solution (10 mL) was added, and the mixture was extracted with 10 mL x 3 EtOAc. The combined organic phases were washed with saturated salt solution (10 mL) and dried over sodium sulfate. After filtration and concentration, the residual was purified by silica gel column chromatography eluted with petroleum ether: ethyl acetate (80: 1-40: 1) to obtain the target product as a colorless oil (333 mg, 32.4%).

¹H NMR (400 MHz, CDCl₃): δ 5.51 (s, 1H), 5.07 (sep, 2H), 2.92 (s, 2H), 2.75 (s, 4H), 1.25 (d, 6H), 1.24 (d, 6H).

### Step 6: diisopropyl 6-(quinolin-4-yl)spiro[3.3]hept-5-ene-2,2-dicarboxylate

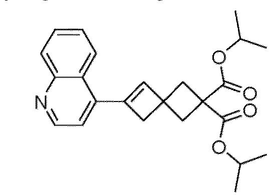

The product (270 mg, 0.65 mmol) of Step 5, 4-quinolinyl-boronic acid (135 mg, 0.78 mmol) and potassium carbonate (180 mg, 1.3 mmol) were placed in a flask, followed by 5 mL of dioxane and tetrakis(triphenylphosphine)palladium (75 mg, 0.065 mmol). Nitrogen was filled for three times and the reaction mixture was stirred at 75°C for 30h under N₂ till the reaction had been completed. The reaction mixture (untreated) was directly used in next step without further purification.

MS ESI: m/z =394.1, [M+H] ⁺.

### Step 7: diisopropyl 6-(quinolin-4-yl)spiro[3.3]heptane-2,2-dicarboxylate

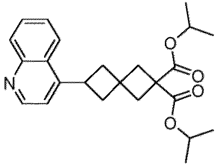

Palladium-carbon catalyst (27 mg, 10% Pd/C) and methanol (10 mL) were added into the reaction mixture of Step 6 and stirred at room temperature for 3 h in hydrogen atmosphere till the reaction had been completed. After filtration and concentration, the residual was purified by silica gel column chromatography eluted with dichloromethane: methanol (100:1-70:1) to obtain the target product as a yellow oil (150 mg, 58% yield in two steps).

MS ESI: m/z =396.1, [M+H] ⁺.

¹H NMR (400 MHz, CDCl₃): δ 8.84 (d, 1H), 8.10-8.12 (m,1H), 7.87-7.89 (m, 1H), 7.68-7.72 (m, 1H), 7.52-7.55 (m, 1H), 7.21 (d, 1H), 5.07 (sep, 2H), 3.96-4.04 (m, 1H), 2.83 (s 2H), 2.66-2.71 (m, 2H), 2.52 (s, 2H), 2.31-2.36 (m, 2H), 1.25 (t, 12H).

### Step 8: 6-(quinolin-4-yl)spiro[3.3]heptane-2,2-dicarboxylic acid

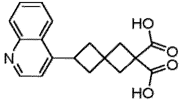

The product of Step 7 (55 mg, 0.14 mmol) was dissolved in ethanol (10 mL) and treated with sodium hydroxide (0.35 mL, 2M). The reaction mixture was heated to 85 °C and stirred for 3 h. Hydrochloric acid (2 M) was added into the solution till the pH of the mixture was 3. The mixture was concentrated to obtain the crude product (70 mg).

MS ESI: m/z =312.0, [M+H] ⁺.

### Step 9: (±)-6-(quinolin-4-yl)spiro[3.3]heptane-2-carboxylic acid

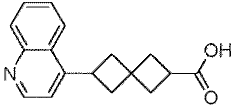

A solution of the crude product (70 mg) of Step 8 in pyridine (10 mL) was heated to reflux 4 h. Hydrochloric acid (2 M) was added into the solution until the pH of the mixture was 3. The mixture was concentrated to obtain the crude product (80 mg).

MS ESI: m/z =268.1, [M+H] ⁺.

### Step 10: (±) N-(4-chlorophenyl)-6-(quinolin-4-yl)spiro[3.3]heptane-2-carboxamide

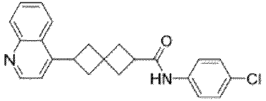

A mixture of the crude product (80 mg) of Step 9, triethylamine (0.076 mL, 0.56 mmol), *N,N-*dimethylformamide (5 mL) and 2-(7-oxide benzotriazole)-N,N,N',N'-HATU (128 mg, 0.336 mmol) was stirred at room temperature for 30 min. p-Chloroaniline (42 mg, 0.336 mmol) was the added into the mixture and stirred at room temperature for 32 h. Ethyl acetate (15 mL) was added, and the mixture was washed with water (50 mL) for three times. The aqueous phase was extracted with ethyl acetate (3 × 10mL. The combined organic phase was washed with brine (5 mL), dried with sodium sulfate, filtered and concentrated. The residual was purified by silica gel column chromatography eluted with dichloromethane: methanol (100:1-70:1) to obtain the crude product (30 mg). The product was further purified by pre-TLC to provide the title product as a yellow solid (12.2 mg; 23% in three steps ).

MS ESI: m/z =377.1, [M+H] ⁺. ¹H NMR (400 MHz, CDCl₃): δ 8.84 (s, 1H), 8.11-8.13 (m,1H), 7.89-7.91 (m, 1H), 7.68-7.72 (m, 1H), 7.50-7.56 (m, 4H), 7.22-7.28 (m, 2H), 3.97-4.06 (m, 2H), 3.05-3.11 (m, 1H), 2.04-2.69 (m, 8H).

### Example 2 (comparative)

### (±)-(cis/trans)-N-(4-chlorophenyl)-6-(quinolin-4-yl)spiro[2.5]octane-1-carboxamide

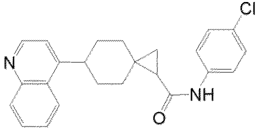

### Step 1: Ethyl 2-(1,4-dioxaspiro[4.5]decan-8-ylidene)acetate

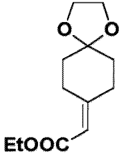

Sodium hydride (52 mg, 0.88 mmol) and triethyl phosphonoacetate (52 mg, 0.83 mmol) were dissolved in *N*, *N*-dimethylformamide (5 mL) in ice bath. After stirring for 0.5 h, a solution of 1, 4-dioxaspiro [4.5] decan-8-ketone (100 mg, 0.65 mmol) in *N*, *N*-dimethylformamide solution (2 mL), was added. After stirring at room temperature for 1h, the mixture quenched with water (50 mL) and extracted with ethyl acetate (20 mL x 3). The combined organic layer was washed with brine and dried over anhydrous sodium sulfate. After filtration and concentration, the residual was purified by silica gel column chromatography eluted with petroleum ether: ethyl acetate (3:1) to obtain the title product as an oil (128 mg, 88%).

¹H NMR (400 MHz, CDCl₃): δ 5.60 (s, 1H), 4.05-4.11 (m, 2H), 3.91-3.92 (d, 4H), 2.95 (t, 3H), 2.31 (t, 3H), 1.67-1.73 (m, 4H), 1.19-1.23 (m, 3H).

### Step 2: (±)-Ethyl 7,10-dioxadispiro[2.2.4⁶.2³]dodecane-1-carboxylate

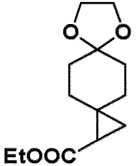

A solution of trimethylsulfonium iodide (193 mg, 0.80 mmol) and potassium tert-butoxide (99 mg, 0.88 mmol) in DMSO (5 mL) was stirred at room temperature for half an hour. The product of Step 1 (60 mg, 0.27 mmol) in DMSO (1 mL) was added. After stirring at room temperature for 2 days, the mixture was quenched with water (50 mL) and extracted with ethyl acetate (20mL x 3). The combined organic layer was washed with brine and dried over anhydrous sodium sulfate. After filtration and concentration, the residual was purified by silica gel column chromatography eluted with petroleum ether: ethyl acetate (5:1) to obtain the product as an oil (46 mg, 43%).

MS ESI: m/z = 241.1, [M+H] ⁺.

### Step 3: (±)-Ethyl 6-oxospiro[2.5]octane-1-carboxylate

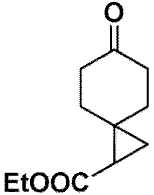

To a solution of the product (100 mg, 0.42 mmol) of Step 2 in tetrahydrofuran (5 mL) was added HCl (5 mL, 3M). After stirring overnight, the mixture was quenched with buffer solution (pH=6, 50 mL) and extracted with ethyl acetate (20mL x 3). The combined organic layer was washed with brine and dried over anhydrous sodium sulfate. After filtration and concentration, the residual was purified by silica gel column chromatography eluted with petroleum ether: ethyl acetate (4: 1) to obtain the product as an oil (86 mg, 105%).

MS ESI: m/z = 197.1, [M+H] ⁺.

### Step 4: (±) Ethyl 6-(((trifluoromethyl)sulfonyl)oxy)spiro[2.5]oct-5-ene-1-carboxylate

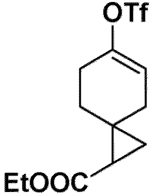

A solution of *N*-phenyl-bis(trifluoromethane)sulfonimide (85 mg, 0.43 mmol) and NaHMDS (0.26 mL, 2M, 0.52 mmol) in tetrahydrofuran (3 mL) was stirred at -78°C under N₂ for 0.5 h. The product of Step 3 (186 mg, 0.5 mmol) was added into the solution. After stirring for 4 h, the mixture was quenched with sodium posphate buffer solution (pH=6, 50 mL) and extracted with ethyl acetate (30mL x 3). The combined organic layer was washed with brine and dried over anhydrous sodium sulfate. After filtration and concentration, the residual was purified by silica gel column chromatography eluted with petroleum ether: ethyl acetate (5:1) to obtain the product as an oil (93 mg, 65%).

¹H NMR (400 MHz, CDCl₃): δ 5.69 (t, 1H), 4.03-4.10 (m, 2H), 2.28-2.20(m, 3H), 1.83-1.97 (m, 3H), 1.51-1.62 (m, 1H), 1.12-1.22 (m, 4H), 0.90-0.94 (m, 1H).

### Step 5: (±)-Ethyl 6-(quinolin-4-yl)spiro[2.5]oct-5-ene-1-carboxylate

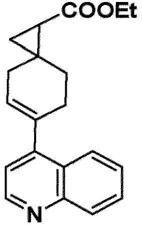

The product (90 mg, 0.28 mmol) of Step 4 and quinoline-4-boric acid (62 mg, 0.36 mmol) were dissolved in tetrahydrofuran (15 mL). Potassium carbonate (114 mg, 0.82 mmol) and tetrakis(triphenylphosphine)palladium (42 mg, 10%) were added into the solution. After at 95°C for 10 h under N₂, the solid was filtered off and the filtrate was treated with buffer salt (pH=6, 100 mL) and extracted with ethyl acetate (50mL x 3). The combined organic layer was washed with brine and dried with anhydrous sodium sulfate. After filtration and concentration, the residual was purified by silica gel column chromatography eluted with petroleum ether: ethyl acetate (5:1) to obtain the product as a yellow oil (30 mg, 36%).

MS ESI: m/z = 308.1, [M+H] ⁺.

### Step 6: (±)-(cis/trans)-Ethyl 6-(quinolin-4-yl)spiro[2.5]octane-1-carboxylate

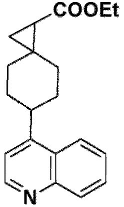

To a solution of the product of Step 5 (460 mg, 1.5 mmol) in ethanol (5 mL) was added palladium-carbon catalyst (46 mg, 10% Pd/C). The reaction mixture was stirred under H₂ overnight. After filtering the palladium-carbon catalyst and concentrating the solution, the residual was purified by silica gel column chromatography eluted with petroleum ether: ethyl acetate (5:1) to obtain the product as a white solid (450 mg, 97%).

MS ESI: m/z = 310.1, [M+H] ⁺.

### Step 7: (±)-(cis/trans)-6-(quinolin-4-yl)spiro[2.5]octane-1-carboxylic acid

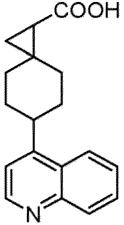

The product (30 mg, 0.1 mmol) of Step 6 was dissolved in tetrahydrofuran/ethanol (2 mL/2 mL)and treated with aqueous lithium hydroxide solution (2M, 2 mL). After stirring at 70 °C for 8 h, the mixture was quenched with phosphate buffer solution (pH=6, 100 mL) and extracted with ethyl acetate (50 mL x 3). The combined organic layer was washed with brine, filtered over anhydrous sodium sulfate and concentrated. The residual was purified by gel column chromatography to obtain two isomers: Isomer A (15 mg) and isomer B (10 mg).

MS ESI: m/z = 282.1, [M+H] ⁺.

### Step 8: (±)-(cis/trans)-N-(4-chlorophenyl)-6-(quinolin-4-yl)spiro[2.5]octane-1-carboxamide

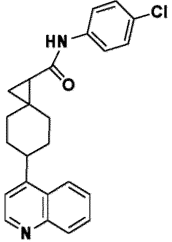

The Isomers A (15 mg, 0.053 mmol) obtained in Step 7, 4-chloroaniline (8.2 mg, 0.064 mmol), 2-(7-oxide benzotriazole)-*N*, *N*, *N*', *N*'-HATU (24 mg, 0.064 mmol) and diisopropylethylamine (28 µL, 0.16 mmol) were dissolved in dichloromethane (5 mL). The resulted solution was stirred at room temperature for 2 h, and then quenched with phosphate buffer solution (pH=6, 100 mL) and extracted with ethyl acetate (50 mLx3). The combined organic layer was washed with brine and dried with anhydrous sodium sulfate. After filtration and concentration, the residual was purified by silica gel column chromatography eluted with petroleum ether: ethyl acetate (1:1) to obtain Example 2A as a white solid (8 mg, 39%).

MS ESI: m/z = 391.1, [M+H] ⁺.

Similarly, Example 2B was obtained from Isomer B of Step 7 as a white solid (9 mg, 44%). MS ESI: m/z = 391.1, [M+H] ⁺.

### Example 3 (comparative)

### (±) N-(4-chlorophenyl)-6-(6-fluoroquinolin-4-yl)spiro[3.3]heptane-2-carboxamide

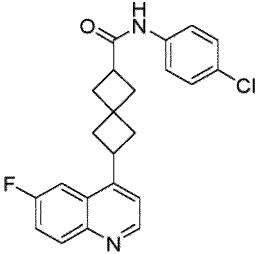

### Step 1: diisopropyl 6-(6-fluoroquinolin-4-yl)-6-hydroxyspiro[3.3]heptane-2,2-dicarboxylate

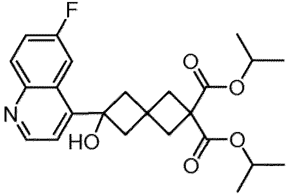

Tert-butyllithium (2.50 mL 1.6 mol/L pentane solution, 4.0 mmol) was slowly added into a of 4-bromine-6-fluoroquinoline (0.4539 g, 2.008 mmol) in 20 mL of THF at -78°C under argon. After stirring for 3 min, a solution of the product (0.5708 g, 2.022 mmol) of step 3 in Example 1 in THF (6 mL) was added dropwise, and the mixture was stirred for 1min and slowly warmed to the room temperature. After acidifying the mixture with acetic acid (0.14 mL) and concentrating, the residual was purified by silica gel column chromatography eluted with petroleum ether: ethyl acetate (0:100-2:3) to obtain the product as a yellow oil (0.43 g, yield: 50%).

MS ESI: m/z =430.2, [M+H] ⁺.

### Step 2: (±) 6-(6-fluoroquinolin-4-yl)spiro[3.3]heptane-2-carboxylic acid

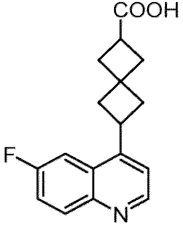

The product (1.48 g, 3.45 mmol) of Step 1 and red phosphorus (0.55 g, 17.8 mmol) were mixed in hydroiodic acid (10 mL, 55%). The mixture was stirred at 140 °C for 4h in a sealed tube, then heated at 180 °C for 20 h. After cooling, to room temperature, red phosphorus was filtered off, and sodium carbonate (2.25 g) was added to neutralize the mixture. Sodium thiosulfate pentahydrate (1.0 g) was added to remove the iodine. Sodium dihydrogen phosphate dihydrate (6.50 g) was added to adjust the pH value. The mixture was extracted with ethyl acetate (25 mL x 3) for three times. The combined organic phases were washed with brine, dried over anhydrous sodium sulfate and concentrated. The residual was purified by silica gel column chromatography eluted with DCM:MeOH (100:0-100:4) to obtain the product as a white solid (1.48 g, yield: 43%).

MS ESI: m/z =286.1, [M+H] ⁺.

### Step 3: (±)-N-(4-chlorophenyl)-6-(6-fluoroquinolin-4-yl)spiro[3.3]heptane-2-carboxamide

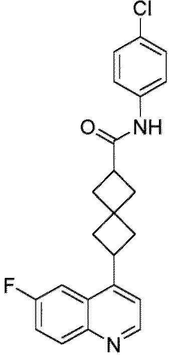

The product (80 mg) of Step 2, triethylamine (0.076 mL, 0.56 mmol), *N,N-*dimethylformamide (5 mL) and HATU (128 mg, 0.336 mmol) were added into a flask. After stirring for 0.5 h, p-chloroaniline (42 mg, 0.336 mmol) was further added, and the mixture was stirred at room temperature for 32 h. Ethyl acetate (15 mL) was added, and the solution was washed with water (50 mL) for three times. The aqueous phase was extracted with ethyl acetate (3x10mL). The combined organic phase was washed with brine (5 mL), dried over sodium sulfate, filtered and concentrated. The residual was purified by silica gel column chromatography eluted with DCM:methanol (100:1-70:1) to obtain the crude product (30 mg) which was further purified by perp-TLC to give the product as a yellow solid (12.2 mg).

MS ESI: m/z =377.1, [M+H] ⁺.

¹H NMR (400 MHz, CDCl₃): δ 8.84 (s, 1 H), 8.11-8.13 (m,1 H), 7.89-7.91 (m, 1 H), 7.68-7.72 (m, 1 H), 7.50-7.56 (m, 4 H), 7.22-7.28 (m, 2 H), 3.97-4.06 (m, 2 H), 3.05-3.11 (m, 1 H), 2.04-2.69 (m, 8H).

### Example 4 (comparative)

### (±)-(cis/trans)-N-(4-chlorophenyl)-6-(6-fluoroquinolin-4-yl)spiro[2.5]octane-1-carboxamide

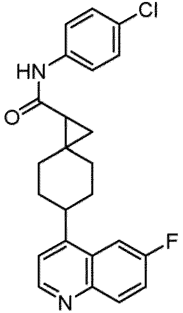

### Step 1: 1,4-dioxaspiro[4.5]dec-7-en-8-yl trifluoromethanesulfonate

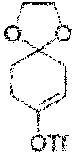

1,4-Cyclohexanedione monoethylene ketal (18.44 g, 118.07 mmol) and N-phenylbis (trifluoromethanesulfonimide) (46.4 g, 129.88 mmol) were dissolved in tetrahydrofuran (200 mL). The mixture was cooled to -78 °C and treated with a solution of NaHMDS solution (71 mL, 2M in THF, 141.68 mmol) over 45 min. After stirring for 1 h, brine (15 mL) was added, and the reaction solution was concentrated. Ethyl acetate (300 mL) was added and the organic layer was washed with 5% sodium hydroxide solution (250 mL) twice. Ethyl acetate was concentrated to obtain the product (27 g; yield: 80%), which was used for the next step without further purification.

¹NMR (400 MHz, CDCl₃): δ 5.66 (t, 1H), 3.99 (d, 4H), 2.54 (s, 2H), 2.40 (s, 2H), 1.91 (t, 2H).

### Step 2: 4,4,5,5-tetramethyl-2-(1,4-dioxaspiro[4.5]dec-7-en-8-yl)-1,3,2-dioxaborolane

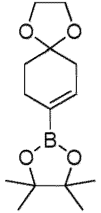

The product (26 g, 83.84 mmol) of Step 1, 4,4,4',4',5,5,5',5'-octamethyl-2,2'-bi(1,3,2-dioxaborolane) (24.5 g, 96.43 mmol), potassium acetate (24.69 g, 251.53 mmol), sodium bromide (3.45 g, 33.53 mmol) and [1,1'-bis (diphenylphosphino) ferrocene] dichloropalladium (3.42 g,4.19 mmol) were added into a two-necked flask containing 1,4-dioxane (300 mL) under N₂. The mixture was stirred at 105 °C for 8 h. After concentration, ethyl acetate (300 mL) was added, and the insoluble substance was filtered off. The residue was purified by silica gel column chromatography eluted with petroleum ether: ethyl acetate (10:0-8:2) to obtain the product as a light yellow oil (15 g, 68%).

¹H NMR (400 MHz, CDCl₃): δ 6.45 (m, 1H), 3.96 (s, 4H), 2.36-2.33(m, 4H), 1.73-1.70 (t, 2H), 1.23 (s, 12H).

### Step 3: 6-fluoro-4-(1,4-dioxaspiro[4.5]dec-7-en-8-yl)quinoline

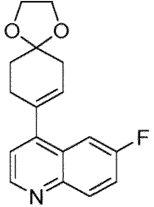

The product (13.5 g,50.72 mmol) of Step 2, 4-bromine-6-fluoroquinoline (10.34 g, 46.11 mmol), potassium carbonate (19.12 g, 138.33 mmol) and tetrakis(triphenylphosphine) palladium were added into a two-necked flask containing 1,4-dioxane (110 mL) and water (30 mL) under N₂. The mixture was stirred at 115 °C for 3 h. After the reaction mixture was concentrated, ethyl acetate (150 mL) was added, and the insoluble substance was filtered off. The residue was purified by silica gel column chromatography eluted with petroleum ether: ethyl acetate (10:0-7:3) to obtain 13 g of the product (yield: 98.9%).

MS ESI: m/z =286.1, [M+H] ⁺.

¹H NMR(400 MHz, CDCl₃): δ 8.81-8.80 (d, 1H), 8.12-8.08 (q, 1H), 7.64-7.62 (q, 1H), 7.49-7.44 (td, 1H), 7.27-7.23(d,1H), 5.76-5.74 (m, 1H), 4.08-4.07 (m, 4H), 2.64-2.60 (m, 2H),2.55-2.54(m, 2H), 2.01-l.98(t, 2H).

### Step 4: 6-fluoro-4-(1,4-dioxaspiro[4.5]decan-8-yl)quinoline

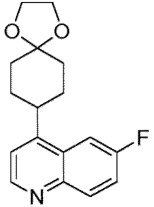

The product (13 g, 45.61 mmol) of Step 3 was placed in a two-necked flask that contained isopropanol (130 mL). Palladium-carbon catalyst (1.3 g, 10%) was added, and the reaction mixture was heated to 55 °e under the atmosphere of hydrogen for 16 h. The reaction mixture was filtered and concentrated to obtain 10 g of the crude product which was used for the next step without further purification.

MS ESI: m/z =288.1, [M+H] ⁺.

### Step 5: 4-(6-fluoroquinolin-4-yl)cyclohexan-1-one

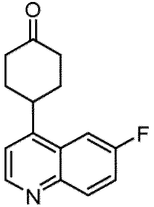

The product (10 g, 34.84 mmol) of Step 4 was placed in a single-neck bottle that contained acetone (100 mL). Hydrochloric acid (30 mL, 4 M) was added and the mixture was attired at 45 °C for 3h. The reaction mixture was concentrated and ethyl acetate (100 mL) and water (100 mL) were added. The pH value of the mixture was adjusted to 9 with saturated sodium bicarbonate aqueous solution. The organic phase was separated and the aqueous phase was further extracted with ethyl acetate (50 mL x 2). The combined ethyl acetate phase was dried with anhydrous sodium sulfate. After filtration and concentration, the residue was purified by silica gel column chromatography eluted with petroleum ether: ethyl acetate (10:0-3:7) to obtain 5.7 g of the product (yield: 74.1%).

MS ESI: m/z =244.1, [M+H] ⁺.

### Step 6: (±) ethyl 2-(4-(6-fluoroquinolin-4-yl)cyclohexylidene)acetate

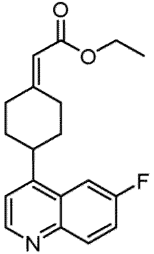

To a suspension of NaH (55 mg, 1.37 mmol) in 5 mL of *N,N*-dimethylformamide cooled in an ice bath under N₂ was added slowly a solution of triethylphosphonoacetate (0.258 mL, 1.3 mmol in 1 mL of *N,N*-dimethylformamide), followed by addition of a solution of the product (0.243 g, 1.00 mmol) of Step 5 in 1 mL of *N,N-*dimethylformamide. After the reaction mixture wad stirred at room temperature for 1h, *N,N-*Dimethylformamide was evaporated and 100 mL of phosphate buffer solution (pH=6) was added. The mixture was extracted with ethyl acetate (3 × 50 mL). The combined organic phase was washed with brine (50 mL) and dried with sodium sulfate. After filtration and concentration, the residue was purified by silica gel column chromatography eluted with petroleum ether: ethyl acetate (100:0-1:1) to obtain the target product.

MS ESI: m/z =314.1, [M+H] ⁺.

### Step 7: (±) (cis/trans) ethyl 6-(6-fluoroquinolin-4-yl)spiro[2.5]octane-1-carboxylate

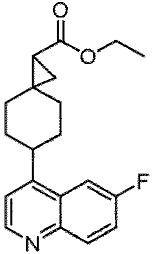

Potassium tert-butoxide (0.097 g, 0.44 mmol) and trimethylsulfonium iodide (0.049 g, 0.44 mmol) were dissolved in 5 mL of dimethyl sulfoxide at room temperature. After stirring overnight, a solution of the product of Step 6 (0.069 g, in 1 mL of DMSO) was added and the reaction mixture was stirred for 2 days at room temperature. The reaction was quenched with 200 mL of water and the mixture was extracted with ethyl acetate (3 x 50mL). The organic phases were washed with brine (50 mL) and dried with sodium sulfate. After filtration and concentration, the residue was purified by silica gel column chromatography eluted with petroleum ether: ethyl acetate (100:0-5:1) to obtain the product.

MS ESI: m/z =328.2, [M+H] ⁺.

### Step 8: (±)-(cis/trans) 6-(6-fluoroquinolin-4-yl)spiro[2.5]octane-1-carboxylic acid

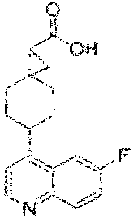

To a solution of the product of Step 7 (0.077 g, 0.23 mmol) in 5 mL of ethyl alcohol at room temperature was added 1mL of LiOH solution (0.198 g, 1N). After stirring at 70 °C for 5 h, water (200 mL) was added and the mixture was extracted with ethyl acetate (3 x 50 mL). The organic phase was washed with brine (50 mL) and dried with sodium sulfate. After filtration and concentration, the residue was purified by silica gel column chromatography eluted with petroleum ether: ethyl acetate (100:0-1:1) to obtain the product.

MS ESI: m/z =300.2, [M+H] ⁺.

### Step 9: (±)-(cis/trans)-N-(4-chlorophenyl)-6-(6-fluoroquinolin-4-yl)spiro[2.5]octane-1-carboxamide

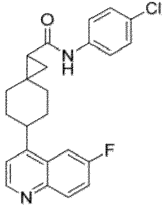

According to the method described in Example **2,** (±) (cis/trans) 6-(quinolone-4-yl) spiro [2.5] octane-1-carboxylic acid in Step 8 was substituted by (±) (cis/trans) 6-(6-fluoroquinoline-4-yl) spiro [2.5] octane-1-arboxylic acid to obtain the title product.

MS ESI: m/z=409.1, [M+H] ⁺.

### Example 5 (±)-(cis/ trans)-N-(4-fluorophenyl)-6-(6-fluoroquinolin-4-yl)spiro[2.5]octane-1-carboxamide (comparative)

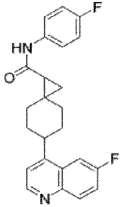

According to the method described in Example 4, 4-chloroaniline in Step 9 was substituted by 4-fluoroaniline to obtain the title product.

MS ESI: m/z=393.2, [M+H] ⁺.

### Example 6 (±)-(cis/trans)-6-(6-fluoroquinolin-4-yl)-N-(4-trifluoromethyl)phenyl)spiro[2.5]octane-1-carboxamide (comparative)

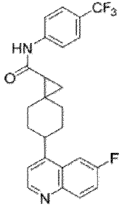

According to the method described in Example 4, the reagent **4**-chloroaniline in Step 9 was substituted by 4-aminotrifluorotoluene to obtain the target compound.

MS ESI: m/z=443.2, [M+H] ⁺.

### Example 7 (±)-(cis/trans)-6-(6-fluoroquinolin-4-yl)-N-phenylspiro[2.5]octane-1-carboxamide (comparative)

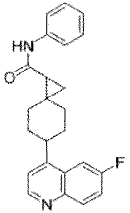

According to the method described in Example 4, the reagent **4**-chloroaniline in Step 9 was substituted by aniline to obtain the target compound.

MS ESI: m/z=375.1, [M+H] ⁺.

### Example 8 (±)-(cis/trans)-4-chloro-N-(6-(6-fluoroquinolin-4-yl)spiro[2.5]octan-1-yl)benzamide (comparative)

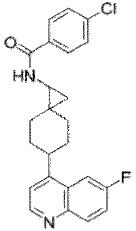

### Step 1: (±)-(cis/trans)-6-(6-fluoroquinolin-4-yl)spiro[2.5]octan-1-amine

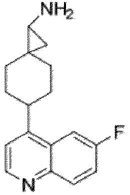

The product (20 mg, 0.067 mmol) of Step 8 in Example **4,** diphenylphosphoryl azide (144 µ L, 0.670 mmol) and triethylamine (19 µ L, 0.13 mmol) were dissolved in toluene under N₂. After stirring at 70 °C for 3 h, the mixture was concentrated, and treated with water/tetrahydrofuran (0.4 mL/0.4 mL) and lithium hydroxide (15 mg, 0.64 mmol). After stirring at room temperature for 30 min, the reaction mixture was extracted with ethyl acetate (3 x 50 mL). The combined organic phase was washed with brine (50 mL), dried with sodium sulfate, filtered and concentrated to obtain the target product.

MS ESI: m/z =270.1, [M+H] ⁺.

### Step 2: (±)-(cis/trans)-4-chloro-N-(6-(6-fluoroquinolin-4-yl)spiro[2.5]octan-1-yl)benzamide

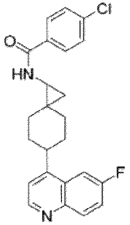

Starting with the product of Step1 and p-chlorobenzoic acid, the title product was obtained according to the method described in Example 2.

MS ESI: m/z =409.1, [M+H] ⁺.

### Example 9 (comparative)

### (±)-(cis/trans)-1-(4-chlorophenyl)-3-(6-(6-fluoroquinolin-4-yl)spiro[2.5]octan-1-yl)urea

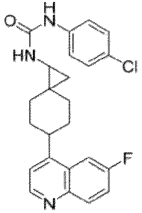

The product (20 mg, 0.074 mmol) of Step 1 in Example **8** and triethylamine (26 µL, 0.15 mmol) were dissolved in 3 mL of methylene chloride. A solution of 4-chlorophenyl isocyanate (14 mg, 0.088 mmol) in 1 mL of DCM was added. After stirring at room temperature for 1 h, water (20 mL) was added, and the mixture was extracted with ethyl acetate (50 mL × 3). The combined organic phase was washed with brine (50 mL) and dried with sodium sulfate. After filtration and concentration, the residual was purified by silica gel column chromatography eluted with petroleum ether:ethyl acetate (100:0-1:1) to obtain the target product.

MS ESI: m/z =424.1, [M+H] ⁺.

### Example10A and 10B (comparative)

### (±) N-(4-chlorophenyl)-7-(6-fluoroquinolin-4-yl)spiro[3.5]nonane-1-carboxamide

### (±) N-(4-chlorophenyl)-7-(6-fluoroquinolin-4-yl)spiro[3.5]nonane-1-carboxamide

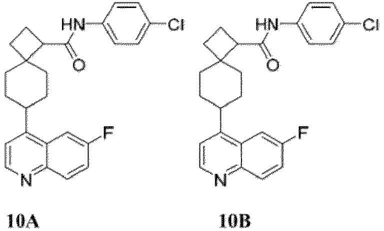

### Step 1: 4-(4-cyclopropylidenecyclohexyl)-6-fluoroquinoline

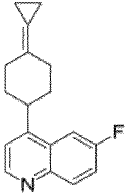

(3-Bromocyclopropyl)triphenylphosphonium bromide (5.72 g, 12.34 mmol) and sodium hydride (592.2 mg, 24.68mmol) were placed in a two-necked flask under N₂. Ultra-dry tetrahydrofuran (15 mL) was added into a reaction flask, and the mixture was heated to 70 °C and stirred for 4 h. A solution of the product (2 g, 8.23 mmol) of Step 5 in Example **4** in tetrahydrofuran (15 mL) was added into the reaction solution. After stirring for 2h, the reaction mixture was cooled to room temperature, and the insoluble substances were filtered off. After concentration, the residual was purified by silica gel column chromatography eluted with petroleum ether: ethyl acetate (10:0-8:2) to obtain the product (white solid, 1.4 g; yield: 63.6%).

MS ESI: m/z =268.1, [M+H] ⁺.

### Step 2: (±) (cis/trans) 7-(6-fluoroquinolin-4-yl)spiro[3.5]nonan-1-one

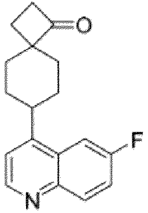

3-Chloroperoxybenzoic acid (370 mg, 2.15 mmol) was added to a solution of the product (500 mg, 1.87 mmol) of Step 1 in dichloromethane (5 mL) dissolved cooled in an ice bath. After stirring for 45 min, methanesulfonic acid (360 mg, 3.74 mmol) was added. After stirring at room temperature for 2 h, the pH of the mixture was adjusted to 9 with saturated aqueous NaHCO₃ solution. The organic phase was washed with brine (10 mLx2), dried with anhydrous sodium sulfate and concentrated. The residual was purified by silica gel column chromatography eluted with petroleum ether: ethyl acetate (100:0-75:25) to obtain the cis-trans isomers **A-1** (160 mg) and **B-1** (200 mg) (yield: 67.9%). MS ESI: m/z =284.1, [M+H] ⁺.

### Step 3: (±)-(cis/trans)-7-(6-fluoroquinolin-4-yl)spiro[3.5]nonane-1-carbonitrile

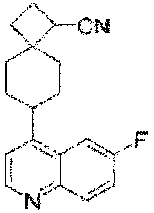

Potassium tert-butoxide (297.4 mg, 2.65 mmol) was added into a solution of cis-trans isomers **A-1** (250 mg, 0.88 mmol) produced in Step 2 in glycol dimethyl ether (10 mL), followed by tosylmethyl isocyanide (344.9 mg, 1.77 mmol) and methanol (42 mg, 1.31 mmol) at 0 °C. After stirring at 0 oC for 10 min and room temperature for 4 h, saturated sodium bicarbonate solution (10 mL) was added. The aqueous phase was extracted with glycol dimethyl ether. The combined organic phase was dried with anhydrous sodium sulfate and concentrated. The residual was purified by silica gel column chromatography eluted with petroleum ether: ethyl acetate (10:0-6:4) to obtain the cis-trans isomer **A-2** (160 mg; yield: 61%).

MS ESI: m/z =295.1, [M+H] ⁺.

Starting with the cis-trans isomer **A-1,** cis-trans isomer **B-2** was prepared by the same method as for cis-trans isomer **A-2.**

MS ESI: m/z =295.1, [M+H] ⁺.

### Step 4: (±)-(cis/trans)-7-(6-fluoroquinolin-4-yl)spiro[3.5]nonane-1-carboxylic acid

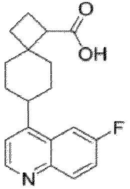

The product **A-2** (150 mg, 0.54 mmol) of Step 3 was added into hydrobromic acid (48%) (4 mL), and the mixture was stirred at 105 °C for two days. The mixture was neutralized with saturated sodium bicarbonate solution till the pH was 4, and extracted with ethyl acetate (15 mLx3). The combined extracts were dried with anhydrous sodium sulfate, concentrated to obtain the product cis-trans isomer **A-3** (147 mg; yield: 92.1%).

MS ESI: m/z =314.1, [M+H] ⁺.

Starting with the cis-trans isomer **B-2**, cis-trans isomer **B-3** was prepared by the same method as for cis-trans isomer **A-3.**

MS ESI: m/z =314.1, [M+H] ⁺.

### Step 5: 10A (±) N (4-chlorophenyl)-7-(6-fluoroquinolin-4-yl)spiro[3.5]nonane-1-carboxamide

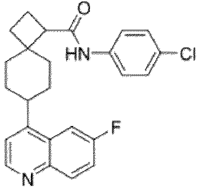

The cis-trans isomer **A-3** (18.0 mg, 0.057 mmol) of Step 4, 4-chloroaniline (8.94 mg, 0.069 mmol), 2-(7-azabenzotriazol-1-yl)-*N,N,N',N'*-tetramethyluronium hexafluorophosphate (26.2 mg, 0.069 mmol) and *N,N*-diisopropylethylamine (22.3 mg, 0.172 mmol) were added into dichloromethane (2 mL). The mixture was stirred at room temperature for 1h, and then at 40 °C for 3 h. Water (10 mL) was added, and the mixture was extracted with dichloromethane (10 mLx3). The combined organic phase was dried with anhydrous sodium sulfate. The residual was purified by silica gel column chromatography eluted with petroleum ether: ethyl acetate (10:0-6:4) to obtain the product (9.06 mg; yield: 37.3%).

MS ESI: m/z =423.1, [M+H] ⁺.

### 10B (±) N-(4-chlorophenyl)-7-(6-fluoroquinolin-4-yl)spiro[3.5]nonane-1-carboxamide

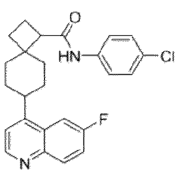

Starting with the cis-trans isomer **B-3**, **10B** was prepared by the same method as for cis-trans isomer **10A.**

MS ESI: m/z =423.1, [M+H] ⁺.

### Example 11A and 11B (comparative) (±) 1-(4-chlorophenyl)-3-(7-(6-fluoroquinolin-4-yl)spiro[3.5]nonan-1-yl)urea

### (±) 1-(4-chlorophenyl)-3-(7-(6-fluoroquinolin-4-yl)spiro[3.5]nonan-1-yl)urea

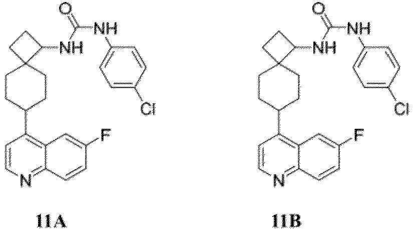

### Step 1: (±) 7-(6-fluoroquinolin-4-yl)spiro[3.5]nonan-1-amine

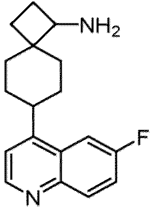

**A3** (20 mg, 0.064 mmol) of Step 4 in Example **10,** diphenylphosphoryl azide (21 mg, 0.077 mmol) and toluene (0.6ml) of triethylamine (7.74 mg, 0.077 mmol) were combined and heated to 70 °C under N₂. After stirring for 2 h, the mixture was concentrated and treated with water/tetrahydrofuran (0.4 mL/0.4 mL) and lithium hydroxide (15 mg, 0.64 mmol) were added. After stirring for 30 min, the mixture was concentrated. Ethyl acetate (20 mL) was added, and the mixture was stirred for 10 min and filtered. The filtrate was dried with anhydrous sodium sulfate and concentrated to obtain the crude product.

MS ESI: m/z =285.2, [M+H] ⁺.

### Step 2: (±) 1-(4-chlorophenyl)-3-(7-(6-fluoroquinolin-4-yl)spiro[3.5]nonan-1-yl)urea

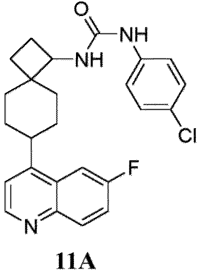

The product (15 mg, 0.053 mmol) of Step 1 and chlorophenyl isocyanate (8.9 mg, 0.058 mmol) were added into tetrahydrofuran (1 mL), and the mixture was stirred at room temperature for 30 min. Water (10 mL) was added, and the mixture was extracted with ethyl acetate (10 mL × 2). The EtOAc extract was dried and concentrated, and the residual was purified by silica gel column chromatography eluted with petroleum ether: ethyl acetate (10:0-6:4) to obtain the product (12.4 mg; yield: 53.9%).

MS ESI: m/z =438.2, [M+H] ⁺.

### 11B (±) 1-(4-chlorophenyl)-3-(7-(6-fluoroquinolin-4-yl)spiro[3.5]nonan-1-yl)urea

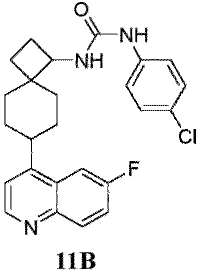

Starting with **A-3, 11B** was prepared by the same method described as for **11A.**

MS ESI: m/z =438.2, [M+H] ⁺.

### Example 12A and 12B (comparative)

### (±) 4-chloro-N-(7-(6-fluoroquinolin-4-yl)spiro[3.5]nonan-1-yl)benzamide

### (±) 4-chloro-N-(7-(6-fluoroquinolin-4-yl)spiro[3.5]nonan-1-yl)benzamide

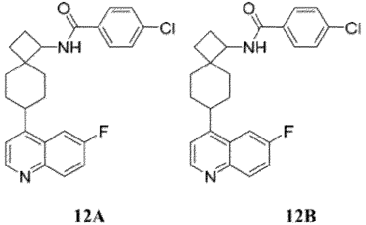

### 12A (±) 4-chloro-N (7-(6-fluoroquinolin-4-yl)spiro[3.5]nonan-1-yl)benzamide

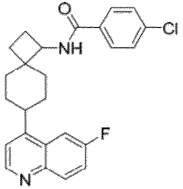

The product (15 mg, 0.053 mmol) of Step 1 in Example **11** and triethylamine (16 mg, 0.158 mmol) were dissolved in tetrahydrofuran (2 mL) cooled in an ice bath. p-Chlorobenzoyl chloride (11.09 mg, 0.063 mmol) was added into the reaction mixture. After stirring at room temperature for 15min, water (10 mL) was added into reaction mixture, and the mixture was extracted with ethyl acetate (10 mL x 3). The combined organic phase was concentrated and the residual was purified by silica gel column chromatography eluted with petroleum ether: ethyl acetate (10:0-6:4) to obtain the product **12A** as a white solid (14.58 mg; yield: 65.7%).

MS ESI: m/z =423.1, [M+H] ⁺.

### 12B (±) 4-chloro-N-(7-(6-fluoroquinolin-4-yl)spiro[3.5]nonan-1-yl)benzamide

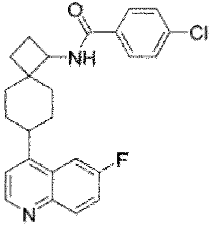

Starting with the product of Step 1 in Example **11B, 12B** was prepared by the same method described as for **12A.**

MS ESI: m/z =423.1, [M+H] ⁺.

### Example 13A and 13B (comparative)

### (±) 4-chloro-N-(7-(6-fluoroquinolin-4-yl)spiro[3.5]nonan-1-yl)benzenesulfonamide

### (±) 4-chloro-N-(7-(6-fluoroquinolin-4-yl)spiro[3.5]nonan-1-yl)benzenesulfonamide

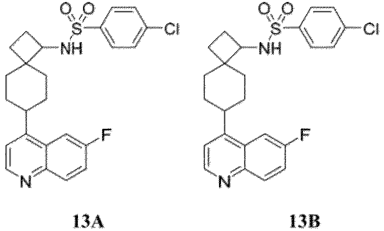

### 13A (±) 4-chloro-N-(7-(6-fluoroquinolin-4-yl)spiro[3.5]nonan-1-yl)benzenesulfonamide

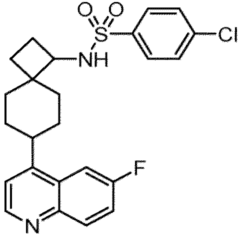

To a solution of the product (13.6 mg, 0.0479 mol) of Step 1 in Example **11A,** 4-dimethylaminopyridine (0.58 mg, 0.005 mmol) and triethylamine (14.5 mg, 0.16 mmol) in tetrahydrofuran (2 mL) cooled in an ice bath was added 4-chlorobenzenesulfonyl chloride (11.1 mg, 0.144 mmol). After stirring at room temperature overnight, water (10 mL) was added and the mixture was extracted with ethyl acetate (10 mL × 3). The combined organic phase was concentrated and the residual was purified by silica gel column chromatography eluted with petroleum ether: ethyl acetate (10:0-6:4) to obtain the product as a white solid (11.8 mg; yield: 53.8%).

MS ESI: m/z =459.1, [M+H] ⁺.

### 13B (±) 4-chloro-N-(7-(6-fluoroquinolin-4-yl)spiro[3.5]nonan-1-yl)benzenesulfonamide

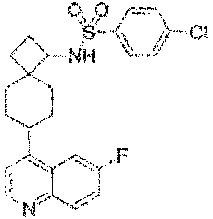

Starting with the product of Step 1 in Example **11B, 13B** was prepared by the same method described as for **13A.**

MS ESI: m/z =459.1, [M+H]⁺.

### Example 14A and 14B (comparative)

### (±) N-(3-bromophenyl)-7-(6-fluoroquinolin-4-yl)spiro[3.5]nonane-1-carboxamide

### (±) N-(3-bromophenyl)-7-(6-fluoroquinolin-4-yl)spiro[3.5]nonane-1-carboxamide

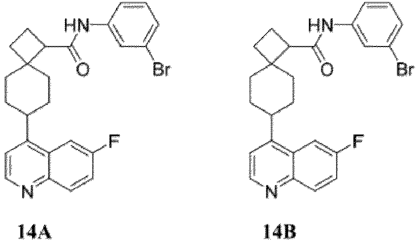

### 14A (±)-N-(3-bromophenyl)-7-(6-fluoroquinolin-4-yl)spiro[3.5]nonane-1-carboxamide

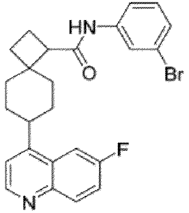

Starting with 3-bromoaniline, **14A** was prepared by the same method described as for **12A.**

MS ESI: m/z =467.1, [M+H] ⁺.

### 14B (±)-N-(3-bromophenyl)-7-(6-fluoroquinolin-4-yl)spiro[3.5]nonane-1-carboxamide

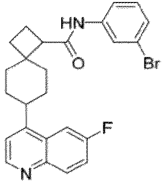

Starting with 3-bromoaniline, **14B** was prepared by the same method described as for **14A.** MS ESI: m/z =467.1, [M+H] ⁺.

### Example 15A and 15B (comparative)

### N-(4-chlorophenyl)-7-(6-fluoroquinolin-4-yl)spiro[3.5]nonane-2-carboxamide

### N-(4-chlorophenyl)-7-(6-fluoroquinolin-4-yl)spiro[3.5]nonane-2-carboxamide

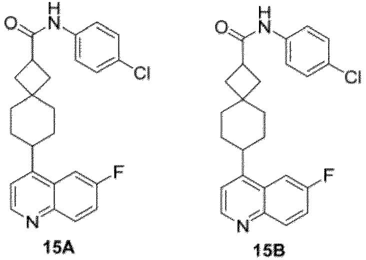

### Step 1 8-methylene-1,4-dioxaspiro[4.5]decane

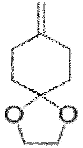

To a suspension of methyltriphenylphosphonium bromide (13.0 g, 83.2 mmol) in diethyl ether (250 mL) cooled at 0 °C as added potassium tert-butoxide (16.6 g, 147.7 mmol) in batches under N₂. After stirring at room temperature for 1 h, a ether solution (30 mL) of 1,4-cyclohexanedione monoethylene ketal was slowly added at 0 °C. The mixture was refluxed for 8 h and then treated with water (100 mL) and filtered. The filtrate was extracted with ethyl acetate (100 mL × 2). The combined organic phase was washed with brine (100 mL), dried with anhydrous sodium sulfate and concentrated to give the title product as a brown oil (10.38 g; yield: 80%).

¹H NMR (400 MHz, CDCl₃): δ 1.70 (t, 4H), 2.28 (t, 4H), 3.97 (s, 4H), 4.67(s, 2H).

### Step 2 8,11-dioxadispiro[3.2.4⁷.2⁴]tridecan-2-one

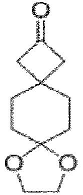

The product (9.7 g, 62.9 mmol) of Step 1 and Zn-Cu couple (55.7 g, 869.0 mmol) were suspended in methyl t-butyl ether (240 mL) under N2. A glycol dimethyl ether solution (60 mL) of trichloroacetic chloride was slowly added at 0 °C. After stirring at room temperature overnight, a methanol solution (300 mL) of saturated ammonia chloride was added at 0 °C and the mixture was stirred at room temperature for 4 h. The mixture was filtered through a pad of celite and filtrate was concentrated. The residual was purified by silica gel column chromatography eluted with a gradient of 0 to 20% ethyl acetate/*n*-hexane to obtain the product as a white solid (8.9 g; yield: 73%).

¹H NMR (400 MHz, CDCl₃): δ 1.66 (t, 4H), 1.82 (t, 4H), 2.79 (s, 4H), 3.96 (s, 4H).

### Step 3 8,11-dioxadispiro[3.2.4⁷.2⁴]tridecane-2-carbonitrile

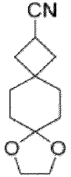

The product (5.0 g, 25.48 mmol) of Step 2, tosylmethyl isocyanide (10.44 g, 53.5 mmol) and anhydrous ethanol (1.8 g, 40.0 mmol) were into a three-necked flask, followed by addition of t-butanol (50 mL) and glycol dimethyl ether (50 mL) cooled at 0 °C, followed by addition of potassium tert-butoxide (12.50 g, 112.1 mmol). After stirring at room temperature overnight, the reaction mixture was poured into ice water (50 mL) and extracted with ethyl acetate (50 mL × 2). The EtOAc extract was washed with brine (50 mL) and dried with anhydrous sodium sulfate. After filtration and concentration, the residual was purified by silica gel column chromatography eluted with a gradient of 0 to 20% ethyl acetate/*n*-hexane to obtain the product as a white solid (2.4 g; yield: 46%).

¹H NMR (400MHz, CDCl₃): δ 3.93(s, 4H), 3.05-2.99(m, 1H), 2.23-2.15(m, 4H), 1.76-1.73(m, 2H), 1.68-1.62(m, 2H), 1.60-1.54(m, 4H).

### Step 4: 7-oxospiro[3.5]nonane-2-carbonitrile

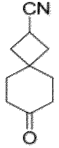

The product (6.88 g, 33.1 mmol) of Step 3 was placed into a round-bottom flask, followed by addition of acetonitrile and water (25 mL-25 mL). The mixture was stirred at 65 °C for 3h. Water (25 mL) was added and the acetonitrile was removed under reduced pressure. The mixture was extracted with ethyl acetate (25 mLx2). The combined organic phase was washed with brine (25 mL), dried with anhydrous sodium sulfate and concentrated. The residual was purified by silica gel column chromatography eluted with a gradient of 0 to 60% ethyl acetate/*n*-hexane to obtain the product (5.0 g; yield: 92%).

¹H NMR (400 MHz, CDCl₃): δ 3.17-3.11(m, 1H), 2.44-2.35(m, 3H), 2.33-2.30(m, 5H), 2.02-1.99(m, 2H), 1.94-1.91(m, 2H).

### Step 5 2-cyanospiro[3.5]non-6-en-7-yl trifluoromethanesulfonate

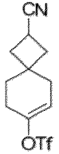

The product (7.0 g, 42.89 mmol) of Step 4 and tetrahydrofuran were added into a three-necked flask under N₂. To the resulted solution cooled to -78 °C was added slowly NaHMDS (25.73 mL, 51.46 mmol, 2M in THF) under N₂. After stirring for at -78 °C, 1h, N-phenylbis (trifluoromethanesulfonimide) (18.40 g in 60 mL of THF) was added slowly and the mixture was stirred continuously for another hour at -78°C. Saturated ammonium chloride solution (10 mL) was added and the mixture was extracted with 50mLx 3 ethyl acetate. The combined organic phase was washed with brine (50 mL) and dried with sodium sulfate. After filtration and concentration, the residual was purified by silica gel column chromatography eluted with a gradient of 0 to 20% ethyl acetate/n-hexane to obtain the product as an oil (12.0 g, yield: 95%).

¹H NMR (400 MHz, CDCl₃): δ 5.67-5.65(m, 1H), 3.15-3.05(m, 1H), 2.39-2.36(m, 3H), 2.30-2.22(m, 5H), 1.92-1.89(t, 1H), 1.83-1.80(t, 1H).

### Step 6: 7-(4,4,5,5-tetramethyl-1,3,2-dioxaborolan-2-yl)spiro[3.5]non-6-ene-2-carbonitrile

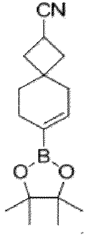

The product (12.0 g, 40.64 mmol) of Step 5, pinacol borate (12.38 g, 48.77 mmol) and potassium acetate (11.97 g, 121.92 mmol) were added into a flask, followed by addition of 1,4-dioxane (150 mL). and [1,1'-bis (diphenylphosphino)ferrocene]dichloropalladium (II) (75 mg, 0.065 mmol) under N₂. The reaction mixture was stirred at 105 °C for 16 h, filtered through a pad of celite and concentrated. The residual was purified by silica gel column chromatography eluted with a gradient of 0 to 15% ethyl acetate/n-hexane to obtain the product (10.8 g; yield: 97.3%).

¹H NMR (400 MHz, CDCl₃): δ 6.43-6.41(m, 1H), 3.06-3.03(m, 1H), 2.25-2.14(m, 8H), 1.68-1.65(m, 1H), 1.61-1.58(m, 1H), 1.26(s, 6H), 1.25(s, 6H).

### Step 7 7-(6-fluoroquinolin-4-yl)spiro[3.5]non-6-ene-2-carbonitrile

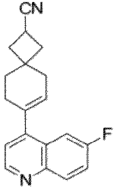

The product (10.8 g, 39.53 mmol) of Step 6, 4-bromo-6-fluoroquinoline (8.94 g, 39.53 mmol), tetrakis(triphenylphosphine)palladium (2.28 g, 1.98 mmol) and cesium carbonate (25.76 g, 79.07 mmol) were added into dioxane (120 mL) and water (30 mL). The mixture was stirred at 100 °C overnight, filtered and concentrated. The residual was purified by silica gel column chromatography eluted with a gradient of 0 to 30% ethyl acetate/n-hexane to obtain the product as a white solid.

MS ESI: m/z =293.2, [M+H] ⁺.

¹H NMR (400 MHz, CDCl₃): δ 8.81(d, 1H), 8.12-8.09(m, 1H), 7.51-7.45(m, 2H), 7.16(s, 1H), 5.75-5.74(m, 1H), 3.23-3.15(m, 1H), 2.43-2.36(m, 8H), 1.99-1.96(m, 2H).

### Step 8: (±)-7-(6-fluoroquinolin-4-yl)spiro[3.5]nonane-2-carboxylic acid

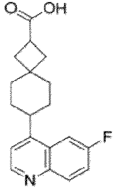

A mixture of the product (11.9 g, 40.73 mmol) of Step 7, water (300 mL), isopropanol (2 mL) and potassium hydroxide (45.6 g, 814 mmol) was stirred at 123 °C for 16h. Hydrochloric acid (12 N) was added until the pH of the mixture was 6.5. Ethanol (150 mL) was added into the reaction mixture, followed by palladium-carbon catalyst (1.0 g) under N₂. The mixture was stirred under H₂ for 16h and filtered through celite. The filtrate was concentrated o obtain the product as a white solid (7.0 g; yield: 80%).

MS ESI: m/z =314.1, [M+H] ⁺.

### Step 9: (±) N (4-chlorophenyl)-7-(6-fluoroquinolin-4-yl)spiro[3.5]nonane-2-carboxamide

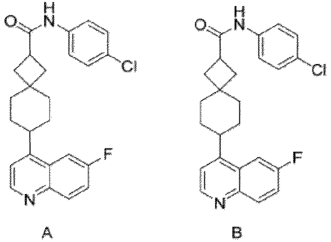

Racemic **15:** A solution of the product (150 mg, 0.48 mmol) of Step 8, 4-chloroaniline (58 mg, 0.48 mol), *N,N-*diisopropylethylamine (185 mg, 1.44 mmol) and HATU (218 mg, 0.57 mmol) in DMF (3.0 mL) was stirred at room temperature for 16 h. The reaction mixture was poured into water (10 mL) and extracted with ethyl acetate (30 mL). The organic phase was washed with saturated sodium bicarbonate (20 mLx2), brine (20 mL), and dried with anhydrous sodium sulfate. After drying and concentration, the residual was purified by silica gel column chromatography eluted with a gradient of 0 to 60% ethyl acetate/n-hexane to obtain racemic **15** (100 mg; yield: 50%).

**Preparation of isomer 15A of Step 9:** 10.0mg of racemic **15** was separated with chiral column AD-H (n-hexane: ethyl alcohol, 80:20). Enantiomer **15A** (5.0 mg; yield: 50%) has a retention time of 33 min.

**Preparation of isomer 15B of Step 9:** 10.0mg of racemic **15** was separated with chiral column AD-H (n-hexane: ethyl alcohol, 80:20). Enantiomer **15B** (5.0 mg; yield: 50%) has a retention time of 39 min.

### Chiral resolution of Example 15

Chiral resolution was performed with Agilent 1260 semi-preparative liquid chromatograph (for axial chirality).

### Resolution of Example 15:

Chiral column: CHIRALPAK AD-H 10 x 250 mm; Flow rate: 3.0mL/min; Detection wavelength: 254nm; Collect two enantiomers.

Conditions: 10 mg sample of Example 15 was dissolved in 6 mL of methanol (injection volume: 0.5mL; eluent: *n*-hexane: ethanol=80: 20 (volume ratio)); The retention times of two enantiomers are 33.0 min **(15A)** and 39.0 min **(15B),** respectively.

### Example 16 (comparative)

### (±) 1-(4-chlorophenyl)-3-(7-(6-fluoroquinolin-4-yl)spiro[3.5]nonan-2-yl)urea

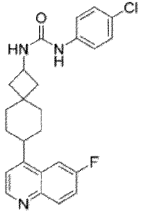

The product (30 mg, 0.096 mmol) of Step 8 in Example **15,** diphenylphosphoryl azide (31.6 mg, 0.115 mmol) and triethylamine (37 mg, 0.288 mmol) were dissolved into toluene (0.5 mL) under N₂ and stirred at 70°C for 2h. *p*-Chloroaniline (37 mg, 0.288 mmol) was added and stirred for 10min. The residual was purified by silica gel column chromatography eluted with petroleum ether: ethyl acetate (10:0-3:2) to obtain the target product (9 mg; yield: 21.4%).

MS ESI: m/z =438.1, [M+H] ⁺.

### Example 17 (comparative)

### (±) 4-chloro-N (7-(6-fluoroquinolin-4-yl)spiro[3.5]nonan-2-yl)benzamide

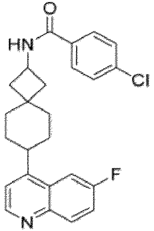

### Step 1: (±)7-(6-fluoroquinolin-4-yl)spiro[3.5]nonan-2-amine

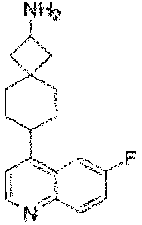

The product (30 mg, 0.096 mmol) of Step 8 in Example **15,** diphenylphosphoryl azide (26 mg, 0.096 mmol) and triethylamine (11.63 mg, 0.115 mmol) were dissolved into toluene (1mL) under the N₂ protection and stirred at 70°C for 2h. After concentration, a mixture of 1 N HCl/THF (2 mL/1 mL) was added and the mixture was stirred at room temperature for 30min. Saturated sodium bicarbonate water solution (10 mL) was added, and the mixture was stirred for 10min and extracted with ethyl acetate (15 mL x 3). Evaporation of EtOAc provided the crude product which was directly used for the next step without further purification.

MS ESI: m/z =285.1, [M+H] ⁺.

### Step 2: (±) 4-chloro-N (7-(6-fluoroquinolin-4-yl)spiro[3.5]nonan-2-yl)benzamide

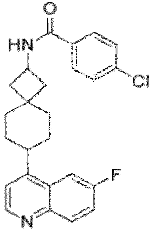

The product (15 mg, 0.0528 mmol) of Step 1 and triethylamine (16 mg, 0.158 mmol) were dissolved into tetrahydrofuran (2 mL). p-Chlorobenzoyl chloride (11.09 mg, 0.0634 mmol) was added into the reaction mixture cooled in ice bath. After stirring for 30min, water (10 mL) was added, and the mixture was extracted with ethyl acetate (10 mL × 3). The ethyl acetate phase was dried, concentrated and the residue was purified with prep-TLC separated (petroleum ether: ethyl acetate = 2:1) to obtain the product (3.55 mg).

MS ESI: m/z =423.1, [M+H] ⁺.

### Example 18 (comparative)

### (±) N (4-bromophenyl)-7-(6-fluoroquinoline-4-yl) spiro [3.5] nonane-2-carboxamide

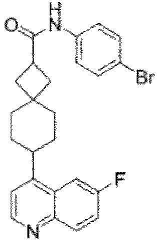

Starting with *p*-bromoaniline, the title compound was prepared by the same method as described in Step 9 of Example **15.**

MS ESI: m/z =467.0, [M+H] ⁺.

### Example 19

### (±) N-(4-fluorophenyl)-7-(6-fluoroquinolin-4-yl)spiro[3.5]nonane-2-carboxamide

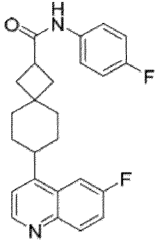

Starting with p-fluoroaniline, the title compound was prepared by the same method as described in Step 9 of Example **15.**

MS ESI: m/z =407.1, [M+H] ⁺.

### Example 20 (comparative)

### (±) 4-bromo-N-(7-(6-fluoroquinolin-4-yl)spiro[3.5]nonan-2-yl)benzamide

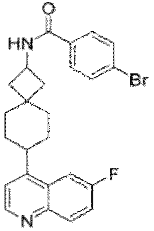

Starting with 4-bromobenzoyl chloride, the title compound was prepared by the same method as described for Example **17.**

MS ESI: m/z =467.0, [M+H] ⁺.

### Example 21 (comparative)

### (±) 4-fluoro-N-(7-(6-fluoroquinolin-4-yl)spiro[3.5]nonan-2-yl)benzamide

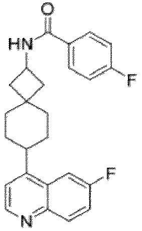

Starting with 4-fluorobenzoyl chloride, the title compound was prepared by the same method as described for Example **17.**

MS ESI: m/z =407.1, [M+H] ⁺.

### Example 22A and 22B (comparative)

### N-(4-chlorophenyl)-2-(6-fluoroquinoline-4-yl) spiro [3.5] nonane-7-carboxamide

### N-(4-chlorophenyl)-2-(6-fluoroquinoline-4-yl) spiro [3.5] nonane-7-carboxamide

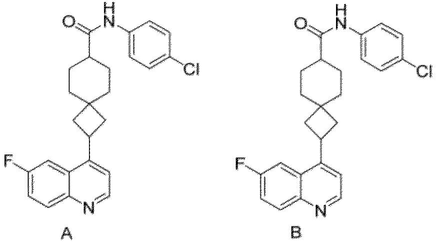

### Step 1: 2-(6-fluoroquinolin-4-yl)-8,11-dioxadispiro[3.2.4⁷.2⁴]tridecan-2-ol

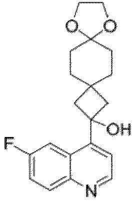

t-Butyllithium (6.38 mL 1.6 M in pentane, 10.2 mmol) was slowly added into a THF solution of 4-bromine-6-fluoroquinoline (1.15 g, 5.1 mmol in 50 mL THF) at -78°C under argon. After stirring for 3 min, a solution of the product Step 2 in Example **15** (1.0 g) in 30 mL of THF was slowly added. After stirring for 10 min, saturated aqueous NH₄Cl solution (30 mL) was added and the mixture was extracted ethyl acetate (3 × 30 mL). The organic phase was washed with brine (30 mL) and dried with anhydrous sodium sulfate. After filtration and concentration, the residual was purified by silica gel column chromatography eluted with petroleum ether: ethyl acetate = (5:1-1:1) to obtain the target product as a brown solid (545 mg; yield: 31.1%).

MS ESI: m/z =344.1, [M+H] ⁺.

¹H NMR (400 MHz, CDCl₃): δ 8.83 (d, 1 H), 8.13 (dd, 1 H), 7.87 (dd, 1 H), 7.48-7.51 (m, 1 H), 7.36 (d, 1 H), 3.92-3.96 (m, 5 H). 2.66-2.69 (m, 2 H), 2.49-2.52 (m, 2 H), 1.99-2.02 (m, 2 H), 1.68-1.71 (m, 2 H), 1.52-1.54 (m, 4 H).

### Step 2: 2-(6-fluoroquinolin-4-yl)-2-hydroxyspiro[3.5]nonan-7-one

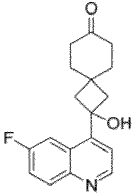

The product (545 mg, 1.59 mmol) of Step 1 was added into 14 mL of 3 mol/L HCl. After stirring at room temperature for 1h, the mixture was extracted with ethyl acetate (3 × 5 mL). The aqueous phase was treated with sodium carbonate aqueous solution to adjust the pH value to 8, and was extracted with ethyl acetate (3 x 15 mL). The organic phase was washed with brine (10 mL), dried over anhydrous sodium sulfate, filtered and concentrated to obtain the target product as a yellow solid (425 mg; yield: 89.5%).

MS ESI: m/z =300.1, [M+H] ⁺.

¹H NMR (400 MHz, CDCl₃): δ 8.83 (d, 1H), 8.15 (dd, 1H), 7.88 (dd, 1H), 7.48-7.53 (m, 1H), 7.37 (d, 1H), 2.80-2.83 (m, 2H), 2.65-2.68 (m, 2H), 2.42-2.45 (m, 2H), 2.24-2.28 (m, 4H), 1.75-1.78 (m, 2H).A proton that can be exchanged by heavy water is not found by nuclear magnetism.

### Step 3: (±) 2-(6-fluoroquinoline-4-yl)-2-hydroxyspiro [3.5] nonane-7-nitrile

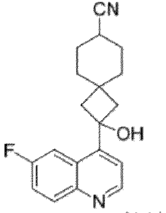

Starting with the product (425 mg, 1.42 mmol) of Step 2, the title product (300 mg; yield: 71.8%) was prepared as a yellow solid by using the same method as described in Step 3 of **Example 10 A and 10B.**

MS ESI: m/z =311.1, [M+H] ⁺.

¹H NMR (400 MHz, CDCl₃): δ 8.81 (d, 1H), 8.13 (dd, 1H), 7.85 (dd, 1H), 7.46-7.51 (m, 1H), 7.33 (d, 1H), 2.48-2.69 (m, 5H), 1.57-1.65 (m, 8H).

### Step 4: (±) 2-(6-fluoroquinoline-4-yl) spiro [3.5] nonane-7-carboxylic acid

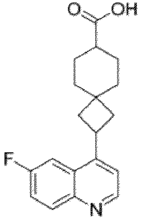

The product (500 mg, 0.97 mmol) of Step 3 and red phosphorus (105 mg, 3.40 mmol) were mixed into 4 mL of concentrated hydroiodic acid (55%). After stirring at 140°C for 2h under N₂, red phosphorus was filtered off and the filtrate was treated with 2.25g of sodium carbonate and then with 1.0 g of sodium thiosulfate pentahydrate to remove iodine. The pH value of the mixture was adjusted to 3 with addition of 1 N NaOH. The solid was collected by filtration washed with petroleum ether to obtain the title product as a yellow solid (170 mg; yield: 56.3%).

MS ESI: m/z =314.1, [M+H] ⁺.

¹H NMR (400 MHz, DMSO-d₆): δ 12.00 (s, 1H), 8.81 (d, 1H), 8.08 (dd, 1H), 7.74 (dd, 1H), 7.63-7.68 (m, 1H), 7.45 (d, 1H), 4.07-4.11 (m, 1H), 2.45-2.49 (m, 1H), 2.30-2.35 (m, 1H), 2.09-2.18 (m, 2H), 1.80-1.99 (m, 3H), 1.64-1.69 (m, 1H), 1.44-1.60 (m, 3H), 1.33-1.40 (m, 2H).

### Step 5:

### 22A N-(4-chlorophenyl)-2-(6-fluoroquinoline-4-yl) spiro [3.5] nonane-7-carboxamide

### 22B N-(4-chlorophenyl)-2-(6-fluoroquinoline-4-yl) spiro [3.5] nonane-7-carboxamide

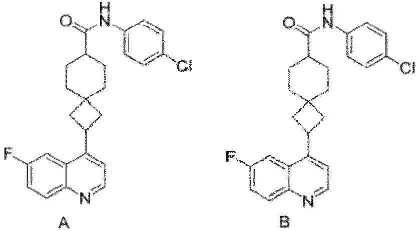

Racemic **22:** The product (30 mg, 0.096 mmol) of Step 4, triethylamine (0.040 mL, 0.288 mmol) and dichloromethane (3 mL) were placed in a flask, followed by HATU (55 mg, 0.144 mmol). After stirring for 1 h, p-chloroaniline (37 mg, 0.288 mmol) was added and the mixture was stirred at room temperature for 24h. After concentration, the residual was purified by silica gel column chromatography eluted with petroleum ether: ethyl acetate = (5:1-1:1) to obtain 30 mg of the crude product, which was washed 3 times with n-hexane to obtain the title compound as a yellow solid (25 mg, yield: 61.1%).

MS ESI: m/z =423.1, [M+H] ⁺.

¹H NMR (400 MHz, DMSO-d₆): δ 9.96 (s, 1H), 8.82 (d, 1H), 8.07-8.10 (m, 1H), 7.76 (d, 1H), 7.63-7.65 (m, 3H), 7.47 (d, 1H), 7. 33 (d, 2H), 4.09-4.13 (m, 1H), 2.21-2.33 (m, 3H), 1.78-2.02 (m, 4H), 1.33-1.63 (m, 6H).

**Preparation of Enantiomer 22A:** 20 mg of Raceme **22** was separated with chiral column AD-H (n-hexane: isopropanol, 70:30). The Enantiomer **22A** (4.57 mg; yield: 22.9%) has a retention time of 12 min.

MS ESI: m/z =423.1, [M+H] ⁺.

¹H NMR (400 MHz, CD₃OD): δ 8.76 (d, 1H), 8.07 (dd, 1H), 7.68 (dd, 1H), 7.55-7.61 (m, 3H), 7.50 (d, 1H), 7.28 (d, 2H), 4.11-4.16 (m, 1H), 2.61-2.67 (m, 1H), 2.28-2.45 (m, 3H), 2.03-2.11 (m, 2H), 1.45-1.90 (m, 7H).

**Preparation of Enantiomer 22B:** 20 mg of Raceme **22** was separated with chiral column AD-H (n-hexane: isopropanol, 70:30). The Enantiomer **22A** (4.67 mg; yield: 23.4%) has a retention time of 20 min.

MS ESI: m/z =423.1, [M+H] ⁺.

¹H NMR (400 MHz, CD₃OD): δ 8.76 (d, 1H), 8.07 (dd, 1H), 7.68 (dd, 1H), 7.55-7.61 (m, 3H), 7.50 (d, 1H), 7.28 (d, 2H), 4.11-4.15 (m, 1H), 2.61-2.67 (m, 1H), 2.28-2.45 (m, 3H), 2.03-2.11 (m, 2H), 1.45-1.90 (m, 7H).

### Resolution of Example 22

Chiral resolution was performed with Agilent 1260 semi-preparative liquid chromatograph.

### Separation of compound in Example 15:

Chiral column: CHIRALPAK AD-H 10*250 mm; Flow rate: 3.0 mL/min; Detection wavelength: 254 nm; two peaks were collected.

Conditions: 20 mg sample in Example 22 was dissolved into 6 mL of methanol (injection volume: 0.5 mL; eluent: n-hexane: isopropanol = 70:30 (volume ratio); The retention times of two enantiomers were 12 min and 20 min, respectively.

### Example 23 (comparative)

### (±) N-(4-bromophenyl)-2-(6-fluoroquinoline-4-yl) spiro [3.5] nonane-7-carboxamide

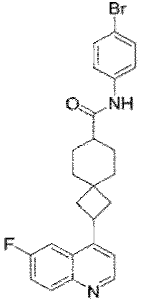

Starting with *p*-bromoaniline, the title compound was prepared by the same method as described for Example **22.**

MS ESI: m/z =467.0, 469.0, [M+H] ⁺.

¹H NMR (400 MHz, CD₃OD): δ 8.77 (d, 1H), 8.07 (dd, 1H), 7.68 (d, 1H), 7.62-7.55 (m, 1H), 7.54-7.47 (m, 3H), 7.43 (d, 2H), 4.19-4.08 (m, 1H), 2.69-2.59 (m, 1H), 2.45-2.36 (m, 1H), 2.36-2.25 (m, 2H), 2.07 (dd, 2H), 1.93-1.84 (m, 1H), 1.84-1.66 (m, 3H), 1.65-1.44 (m, 3H).

### Example 24 (comparative)

### (±) N-(4-fluorophenyl)-2-(6-fluoroquinoline-4-yl) spiro [3.5] nonane-7-carboxamide

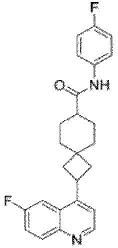

Starting with p-fluoroaniline, the title compound was prepared by the same method as described for Example **22.**

MS ESI: m/z =407.1, [M+H] ⁺.

1H NMR (400 MHz, CD₃OD): δ 8.76 (d, 1H), 8.07 (dd, 1H), 7.68 (dd, 1H), 7.62-7.47 (m, 4H), 7.03 (t, 2H), 4.13 (dt, 1H), 2.68-2.60 (m, 1H), 2.46-2.38 (m, 1H), 2.38-2.25 (m, 2H), 2.07 (dd, 2H), 1.94-1.82 (m, 1H), 1.82-1.64 (m, 3H), 1.64-1.44 (m, 3H).

### Example 25

### N-(4-fluorophenyl)-7-(6-fluoroquinolin-4-yl)spiro[3.5]nonane-2-carboxamide

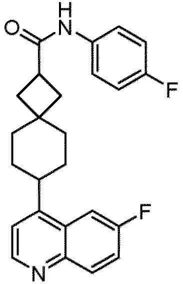

### Step 1: (S)-1-phenylethyl (±)-(7-(6-fluoroquinolin-4-yl)spiro[3.5]nonane-2-carboxylate

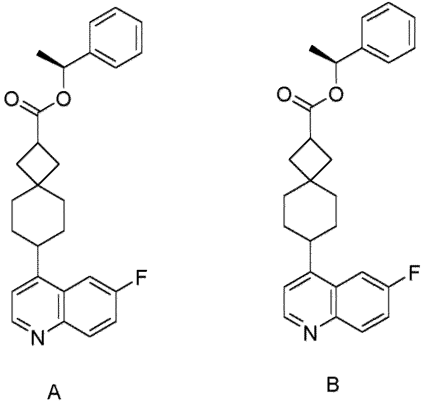

The product (7.0 g, 22.36 mmol) of Step 8 in Example 15 was dissolved into THF (110 mL), followed addition of by dicyclohexylcarbodiimide (5.54 g, 26.84 mmol) and 4-dimethylaminopyridine (2.73 g, 22.36 mmol). After stirring at room temperature for 15min, (*S*)-1-phenylethanol (3.27 g, 26.84 mmol) was added into the reaction mixture. The mixture was stirred for 24h and the byproduct dicyclohexylurea was filtered off. The filtrate was concentrated and the residual was purified by silica gel column chromatography for purification elute with a gradient of 0-25% ethyl acetate/n-hexane to obtain the title compound as a colorless oil (7.0 g; yield: 75%).

MS ESI: m/z =417.1, [M+H] ⁺.

### Separation (S)-1-phenylethyl (±)7-(6-fluoroquinoline-4-yl) spiro [3.5] nonane-2-carboxylic ester

Separation performed with Agilent 1260 semi-preparative liquid chromatograph system.

Chiral column: DAICEL IC 30 x 250 mm, 5 µm; Flow rate: 1.0 mL/min; Eluent: n-hexane: Ethanol=80: 20 (volume ratio); Detection wavelength: 254 nm; two peaks were collected at 10.89 min (isomer **A)** and 12.52 min (isomer **B)** respectively.

The product of Step 1 (7 g) was separated under the above-mentioned conditions to provide isomer **A** (2.98 g) and isomer **B** (2.55g).

### Step 2: (-)-7-(6-fluoroquinoline-4-yl) spiro [3.5] nonane-2-carboxylic acid

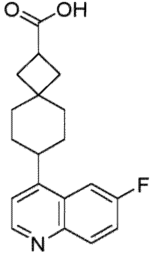

Isomer A in Step 1 was dissolved in methanol (5.0 mL). Under N₂, palladium hydroxide carbon catalyst (70 mg, 10%) and pure water (0.08 mL) were added and the mixture was stirred at room temperature under H₂ for 16h. Palladium catalyst was filtered off through celite. The filtrate was concentrated and the residual was purified by silica gel column chromatography for purification eluted with a gradient of 0-10% methanol/ dichloromethane to obtain the title product as a white solid (340.0 mg; yield: 90.0%), [α]_{D}²⁵= -31.8448 (c = 0.50, CHCl₃).

MS ESI: m/z =314.1, [M+H] ⁺.

### Step 3: N-(4-fluorophenyl)-7-(6-fluoroquinoline-4-yl) spiro [3.5] nonane-2-carboxamide

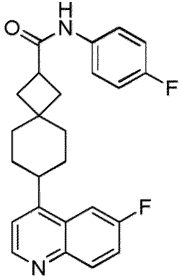

The product (30 mg, 0.096 mmol) of Step 2, 4-fluoroaniline (24 mg, 0.19 mol), 1-ethyl-(3-dimethyllaminopropyl) carbonyl diamide hydrochloride (37 mg, 0.19 mmol) and 1-hydroxybenzotriazole (19 mg, 0.14 mmol) were added into dichloromethane (5.0 mL). Triethylamine (29 mg, 0.29 mmol) was then added, and the mixture was stirred at room temperature for 16 h in an ice bath. The reaction mixture was poured into water (10 mL) and extracted with ethyl acetate (30 mL). The organic phase was washed with saturated sodium bicarbonate (20 mL x 2), brine (20 mL) and dried with anhydrous sodium sulfate. After filtration and concentration, the residual was purified by silica gel column chromatography eluted with a gradient of 0-60% ethyl acetate/n-hexane to obtain the target product (30 mg; yield: 74%).

MS ESI: m/z =407.1, [M+H] ⁺.

¹H NMR (400 MHz, CDCl₃):8 8.80(d, 1H), 8.13-8.10(dd, 1H), 7.67-7.64(dd, 1H), 7.52-7.45(m, 3H), 7.28-7.27(m, 1H), 7.04-7.00(m, 3H),3.15-3.07(m, 2H), 2.27-2.20(m, 3H),2.11-2.07(m, 1H), 2.04-1.99(m, 2H),1.96-1.93(m, 1H), 1.87-1.85(m, 1H), 1.71-1.61(m, 4H).

### Example 26 (comparative)

### N-(4-bromophenyl)-7-(6-fluoroquinoline-4-yl) spiro [3.5] nonane-2-carboxamide

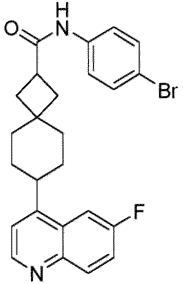

Using p-bromoaniline and the product of Step 2 of Example **25,** the title product was prepared by the same method as described in Step 3 for Example **25.**

MS ESI: m/z =487.1, [M+H] ⁺.

¹H NMR (400 MHz, CDCl₃):8 8.80(d, 1H), 8.13-8.10(dd, 1H), 7.67-7.64(dd, 1H), 7.50-7.44(m, 5H), 7.28-7.27(m, 1H), 7.04(s,1H),3.13-3.09(m, 2H), 2.23-2.20(m, 3H), 2.11-2.07(m, 1H), 2.04-1.92(m, 2H),1.96-1.92(m, 1H), 1.88-1.85(m, 1H), 1.71-1.59(m, 4H).

### Example 27 (comparative)

### (7-(6-fluoroquinoline-4-yl)-N (pyridine-2-yl) spiro [3.5] nonane-2-carboxamide

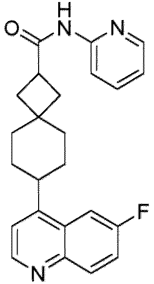

The produt (30 mg, 0.096 mmol) of Step 2 in Example **25,** 2-aminopyridine (11.6 mg, 0.12 mol) and *N*-methylimidazole (16.6 mg, 0.20 mmol) were mixed in MeCN (2.0 mL), followed by addition of *N,N,N',N'*-tetramethylchloroformamidinium hexafluorophosphate (32 mg, 0.115 mmol). The mixture was stirred at room temperature for 16h and was poured into water (10 mL) and extracted with ethyl acetate (30 mL). The organic phase was washed with saturated sodium bicarbonate (20 mL x 2) and brine (20 mL) and dried it with anhydrous sodium sulfate. After filtration and concentration, the residual was purified by silica gel column chromatography eluted with a gradient of 0-60% ethyl acetate/n-hexane to obtain the target product (35 mg; yield: 86%).

MS ESI: m/z =390.1, [M+H] ⁺.

¹H NMR (400 MHz, CDCl₃): δ 8.80(d, 1H), 8.27-8.24(t, 2H), 8.13-8.10(dd, 1H), 7.77(s, 1H), 7.73-7.69(t, 1H), 7.67-7.64(dd, 1H), 7.50-7.44(td, 1H), 7.28-7.27(m, 1H), 7.05-7.02(t, 1H), 3.19-3.12(m, 2H), 2.27-2.22(m, 3H), 2.14-2.09(m, 1H),2.06-1.99(m, 2H), 1.96-1.93(m, 1H), 1.88-1.84(m, 1H), 1.72-1.60(m, 4H).

### Example 28 (comparative)

### N-(3-fluoro-4-(1-methyl-1H-pyrazol-3-yl)phenyl)-7-(6-fluoroquinolin-4-yl)spiro[3.5]nonane-2-carboxamide

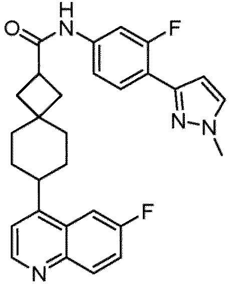

Using 3-fluoro-4-(1-methyl-1*H*-pyrazol-yl) aniline and the product of Step 2 of Example **25,** the title product was prepared by the same method as described in Step 3 for Example **25.**

MS ESI: m/z =487.1, [M+H] ⁺.

¹¹H NMR (400 MHz, CDCl₃):8 8.80(d, 1H), 8.14-8.10(dd, 1H),7.81(s, 1H), 7.74(d, 1H), 7.67-7.62(td, 2H), 7.50-7.45(td, 2H), 7.28-7.27(m, 1H), 7.14(d, 1H), 7.09(s, 1H), 3.95(s, 3H), 3.14-3.10(m, 2H), 2.28-2.21(m, 3H), 2.13-2.08(m, 1H), 2.05-2.00(m, 2H), 1.96-1.93(m, 1H), 1.88-1.85(m, 1H), 1.72-1.60(m, 4H).

### Example 29 (comparative)

### N-(2-chlorophenyl)-7-(6-fluoroquinolin-4-yl)spiro[3.5]nonane-2-carboxamide

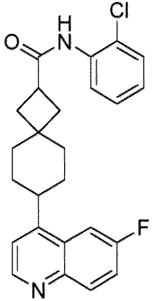

Using 2-chloroaniline and the product of Step 2 of Example **25,** the title product was prepared by the same method as described in Step 3 for Example **25.**

MS ESI: m/z = 423.0 [M+H] ⁺.

¹H NMR (400 MHz, CDCl₃) δ 8.81 (d, 1H), 8.44 (d, 1H), 8.12 (dd, 1H), 7.66 (dd, 1H), 7.61 (s, 1H), 7.47 (ddd, 1H), 7.37 (dd, 1H), 7.33-7.26 (m, 2H), 7.04 (td, 1H), 3.21 (p, 1H), 3.16-3.07 (m, 1H), 2.33-2.19 (m, 3H), 2.18-2.10 (m, 1H), 2.09-2.02 (m, 1H), 2.02-1.90 (m, 2H), 1.90-1.81 (m, 1H), 1.74-1.66 (m, 1H), 1.65-1.55 (m, 3H).

### Example 30 (comparative)

### N-(4-chloro-2-fluorophenyl)-7-(6-fluoroquinoline-4-yl) spiro [3.5] nonane-2-carboxamide

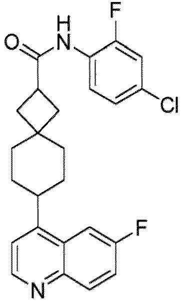

Using 4-chloro-2-fluoroaniline and the product of Step 2 of Example **25,** the title product was prepared by the same method as described in Step 3 for Example **25.**

MS ESI: 441.1 [M+H] ⁺.

¹H NMR (400 MHz, CDCl₃) δ 8.81 (d, 1H), 8.36 (t, 1H), 8.12 (dd, 1H), 7.65 (dd, 1H), 7.47 (ddd, 1H), 7.27 (d, 1H), 7.22 (s, 1H), 7.16-7.13 (m, 1H), 7.13-7.10 (m, 1H), 3.23-3.07 (m, 2H), 2.30-2.18 (m, 3H), 2.16-2.08 (m, 1H), 2.07-1.90 (m, 3H), 1.90-1.82 (m, 1H), 1.73-1.58 (m, 4H).

### Example 31 (comparative)

### N-(3-fluorophenyl)-7-(6-fluoroquinoline-4-yl) spiro [3.5] nonane-2-carboxamide

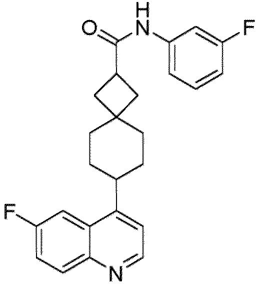

Using 3-fluoroaniline and the product of Step 2 of Example **25,** the title product was prepared by the same method as described in Step 3 for Example **25.**

MS ESI: m/z =407.1, [M+H] ⁺.

¹H NMR (400 MHz, CDCl₃): δ 8.81 (d, 1H), 8.10-8.14 (m, 1H), 7.65 (dd, 1H), 7.45-7.55 (m, 2H), 7.27-7.29 (m, 2H), 7.11-7.16 (m, 2H), 6.80-6.83 (m, 1H), 3.10-3.14 (m, 2H), 2.20-2.27 (m, 3H), 2.07-2.12 (m, 1H), 1.85-2.01 (m, 4H), 1.56-1.71 (m, 4H).

### Example 32 (comparative)

### N-(3-bromophenyl)-7-(6-fluoroquinoline-4-yl) spiro [3.5] nonane-2-carboxamide

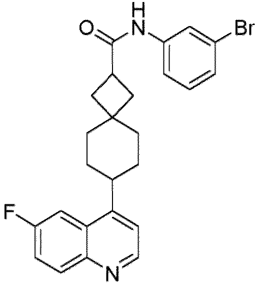

Using 3-bromaniline and the product of Step 2 of Example **25,** the title product was prepared by the same method as described in Step 3 for Example **25.**

MS ESI: m/z =467.0, [M+H] ⁺.

¹H NMR (400 MHz, CDCl₃): δ 8.81 (d, 1H), 8.12-8.15 (m, 1H), 7.84 (s, 1H), 7.65 (dd, 1H), 7.42-7.50 (m, 2H), 7.27-7.29 (m, 1H), 7.16-7.23 (m, 2H), 7.03 (s, 1H), 3.10-3.13 (m, 2H), 2.20-2.27 (m, 3H), 2.07-2.12 (m, 1H), 1.85-2.07 (m, 4H), 1.56-1.71 (m, 4H).

### Example 33 (comparative)

### 7-(6-fluoroquinoline-4-yl)-N-(pyridine-3-yl) spiro [3.5] nonane-2-carboxamide

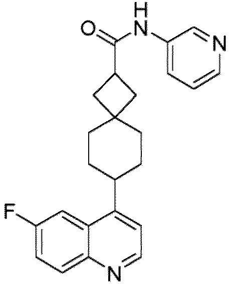

Using 3-aminopyridine and the product of Step 2 of Example **25,** the title product was prepared by the same method as described in Step 3 for Example **25.**

MS ESI: m/z =390.0, [M+H] ⁺.

¹H NMR (400 MHz, CDCl₃): δ 8.81 (d, 1H), 8.59 (s, 1H), 8.36 (s, 1H), 8.27 (d, 1H), 8.12-8.16 (dd, 1H), 7.66 (dd, 1H), 7.46-7.51 (m, 1H), 7.29-7.32 (m, 2H), 7.20 (s, 1H), 3.10-3.19 (m, 2H), 2.22-2.29 (m, 3H), 2.10-2.15 (m, 1H), 1.86-2.06 (m, 4H), 1.56-1.71 (m, 4H).

### Example 34 (comparative)

### N-(3-fluoro-4-chlorophenyl)-7-(6-fluoroquinoline-4-yl) spiro [3.5] nonane-2-carboxamide

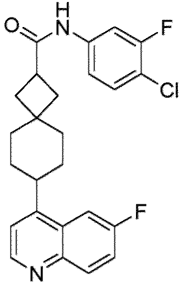

Using 3-fluoro-4-chloroaniline and the product of Step 2 of Example **25,** the title product was prepared by the same method as described in Step 3 for Example **25.**

MS ESI: m/z=441.1, [M+H] ⁺.

### Example 35 (comparative)

### 7-(6-fluoroquinolin-4-yl)-N-(4-(trifluoromethyl)phenyl)spiro[3.5]nonane-2-carboxamide

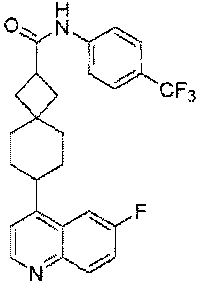

Using 4-trifluoromethyl phenylamine and the product of Step 2 of Example **25,** the title product was prepared by the same method as described in Step 3 for Example **25.**

MS ESI: m/z=457.0, [M+H] ⁺.

1H NMR (400 MHz, CDCl₃) δ 8.81 (d, 1H), 8.13 (dd, 1H), 7.64-7.68 (m, 3H), 7.59 (s, 1H), 7.57 (s, 1H), 7.48 (ddd, 1H), 7.28 (d, 1H), 7.22 (s, 1H), 3.09-3.19 (m, 2H), 2.22-2.28 (m, 3H), 2.11 (t, 1H), 1.99-2.06 (m, 2H), 1.92-1.96 (m, 1H), 1.85-1.88 (m, 1H), 1.68-1.72 (m, 1H), 1.63-1.66 (m, 1H), 1.58-1.62 (m, 2H).

### Example 36 (comparative)

### N-(3-chlorophenyl)-7-(6-fluoroquinoline-4-yl) spiro [3.5] nonane-2-carboxamide

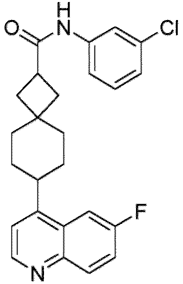

Using 3-chloroaniline and the product of Step 2 of Example **25,** the title product was prepared by the same method as described in Step 3 for Example **25.**

MS ESI: m/z=422.9, [M+H] ⁺.

### Example 37 (comparative)

### N-(3-fluoro-4-bromophenyl)-7-(6-fluoroquinoline-4-yl) spiro [3.5] nonane-2-carboxamide

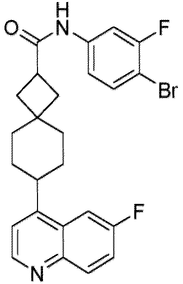

Using 3-fluoro-4-bromaniline and the product of Step 2 of Example **25,** the title product was prepared by the same method as described in Step 3 for Example **25.**

MS ESI: m/z=485.4, [M+H] ⁺.

### Example 38 (comparative)

### N-(3-chloro-4-fluorophenyl)-7-(6-fluoroquinoline-4-yl) spiro [3.5] nonane-2-carboxamide

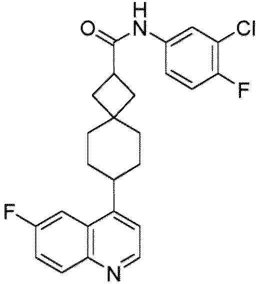

Using 3-chloro-4-fluoroaniline and the product of Step 2 of Example **25,** the title product was prepared by the same method as described in Step 3 for Example **25.**

MS ESI: m/z=441.1, [M+1] ⁺.

¹H NMR (400 MHz, CDCl₃) δ 8.81 (d, 1H), 8.11 (dd, 1H), 7.76 (dd, 1H), 7.65 (dd, 1H), 7.51-7.43 (m, 1H), 7.36-7.30 (m, 1H), 7.28 (s, 1H), 7.09 (t, 1H), 7.02 (s, 1H), 3.10 (p, 2H), 2.26-2.18 (m, 3H), 2.14-2.06 (m, 1H), 2.05-1.97 (m, 2H), 1.96-1.90 (m, 1H), 1.89-1.83 (m, 1H),1.74-1.57 (m, 4H).

### Example 39 (comparative)

### (2S,4s,7S)-7-(6-fluoroquinoline-4-yl)-N-(3-(trifluoromethyl) phenyl) spiro [3.5] nonane-2-carboxamide

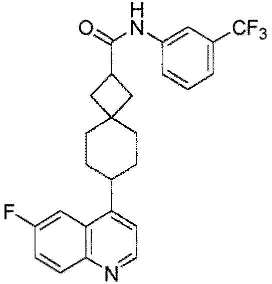

Using 3-(trifluoromethyl)aniline and the product of Step 2 of Example **25,** the title product was prepared by the same method as described in Step 3 for Example **25.**

MS ESI: m/z=457.1, [M+1] ⁺.

¹H NMR (400 MHz, CDCl₃) δ 8.81 (d, 1H), 8.11 (dd, 1H), 7.88 (s, 1H), 7.72 (d, 1H), 7.65 (dd, 1H), 7.50-7.40 (m, 2H), 7.36 (d, 1H), 7.25 (s, 1H),7.14 (s, 1H), 3.13 (p, 2H), 2.29-2.19 (m, 3H), 2.15-2.07 (m, 1H), 2.06-1.98 (m, 2H), 1.97-1.90 (m, 1H), 1.89- 1.83 (m, 1H), 1.73-1.57 (m, 4H).

### Example 40 (comparative)

### N-(3, 4-difluorophenyl)-7-(6-fluoroquinoline-4-yl) spiro [3.5] nonane-2-carboxamide

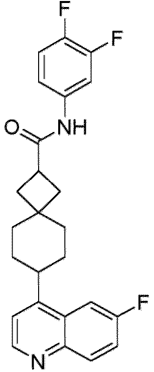

Using 3, 4-difluroaniline and the product of Step 2 of Example **25,** the title product was prepared by the same method as described in Step 3 for Example **25.**

MS ESI: m/z=425.1, [M+H] ⁺.

¹H NMR (400 MHz, CDCl₃): δ 8.81 (d, 1H), 8.12 (dd, 1H), 7.70-7.62 (m, 2H), 7.50-7.44 (m, 1H), 7.28 (s, 1H), 7.08 (d, 1H), 7.01 (s, 1H), 3.16-3.05 (m, 2H), 2.27-2.19 (m, 3H), 2.13-2.06 (m, 1H), 2.06-1.98 (m, 2H), 1.97-1.91 (m, 1H), 1.89-1.83 (m, 1H), 1.73-1.64 (m, 2H), 1.64-1.57 (m, 2H).

### Example 41 (comparative)

### 7-(6-fluoroquinoline-4-yl)-N-(2-(trifluoromethyl) phenyl) spiro [3.5] nonane-2-carboxamide

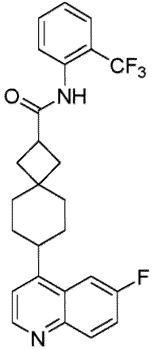

Using 2-(trifluoromethyl)aniline and the product of Step 2 of Example **25,** the title product was prepared by the same method as described in Step 3 for Example **25.**

MS ESI: m/z=457.1, [M+H] ⁺.

¹H NMR (400 MHz, CDCl₃): δ 8.81 (d, 1H), 8.27 (d, 1H), 8.12 (dd, 1H), 7.65 (dd, 1H), 7.61 (d, 1H), 7.57 (t, 1H), 7.47 (ddd, 1H), 7.39 (s, 1H), 7.28 (d, 1H), 7.23 (t, 1H), 3.23-3.15 (m, 1H), 3.15-3.08 (m, 1H), 2.31-2.23 (m, 1H), 2.23-2.18 (m, 1H), 2.18-2.11 (m, 1H), 2.09-2.03 (m, 1H), 1.99-1.91 (m, 2H), 1.89-1.83 (m, 1H), 1.73-1.64 (m, 2H), 1.64-1.59 (m, 2H), 1.58-1.52 (m, 1H).

### Example 42 (comparative)

### N-(5-chloropyridine-2-yl)-7-(6-fluoroquinoline-4-yl) spiro [3.5] nonane-2-carboxamide

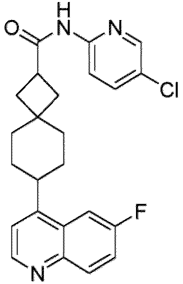

Using 2-amino-5-chloropyridine and the product of Step 2 of Example **25,** the title product was prepared by the same method as described for Example **27.**

MS ESI: m/z =424.1, [M+H] ⁺.

¹H NMR (400 MHz, CDCl₃) δ 8.81 (d, 1H), 8.23 (dd, 2H), 8.12 (dd, 1H), 7.82 (s, 1H), 7.70 -7.63 (m, 2H), 7.47 (ddd, 1H), 7.27 (d, 1H), 3.22-3.08 (m, 2H), 2.21-2.26 (m, 3H), 2.14-2.09 (m, 1H), 2.07-2.01 (m, 1H), 1.99-1.93 (m, 3H), 1.88-1.85 (dt, 1H), 1.71-1.54 (m, 3H).

### Example 43 (comparative)

### N-([1,1'-biphenyl]-4-yl)-7-(6-fluoroquinolin-4-yl)spiro[3.5]nonane-2-carboxamide

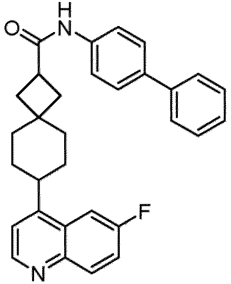

Using [1,1'-biphenyl]-4-amine and the product of Step 2 of Example **25,** the title product was prepared by the same method as described in Step 3 for Example **25.**

MS ESI: M/Z=465.1, [M+1] ⁺.

¹H NMR (400 MHz, CDCl₃): δ 8.81 (d, 1H), 8.12 (dd, 1H), 7.68-7.61 (m, 3H), 7.57 (dd, 4H), 7.50-7.46 (m, 1H), 7.43 (t, 2H), 7.33 (t, 1H), 7.28 (d, 1H), 7.12 (s, 1H), 3.14 (m, 2H), 2.30-2.19 (m, 3H), 2.11 (m, 1H), 2.03(m, 2H), 1.95 (dd, 1H), 1.87 (d, 1H), 1.74-1.59 (m, 4H).

### Example 44 (comparative)

### N-([1,1'-biphenyl]-3-yl)-7-(6-fluoroquinolin-4-yl)spiro[3.5]nonane-2-carboxamide

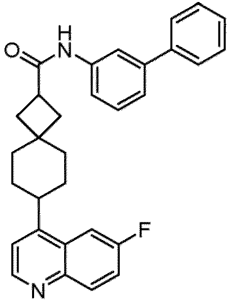

Using [1,1'-biphenyl]-3-amine and the product of Step 2 of Example **25,** the title product was prepared by the same method as described in Step 3 for Example **25.**

MS ESI: M/Z=465.1, [M+1] ⁺.

¹H NMR (400 MHz, CDCl₃): δ 8.81 (d, 1H), 8.12 (dd, 1H), 7.85 (s, 1H), 7.66 (dd, 1H), 7.62-7.58 (m, 2H), 7.52-7.32 (m, 7H), 7.28 (d, 1H), 7.12 (s, 1H), 3.20-3.08 (m, 2H), 2.31-2.21 (m, 3H), 2.09 (m, 2H), 1.97-1.83 (m, 3H), 1.75-1.59 (m, 4H).

### Example 45 (comparative)

### N-(3-chloro-4-(trifluoromethyl) phenyl)-7-(6-fluoroquinoline-4-yl) spiro [3.5] nonane-2-carboxamide

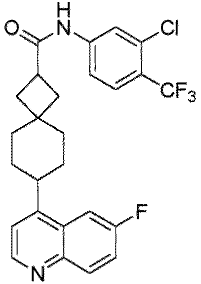

Using 3-chlorine-4-trifluoromethyl-aniline and the product of Step 2 of Example **25,** the title product was prepared by the same method as described in Step 3 for Example **25.**

MS ESI: m/z =491.0, [M+H] ⁺.

¹H NMR (400 MHz, CDCl₃) δ 8.81 (d, 1H), 8.12 (dd, 1H), 7.86 (s, 1H), 7.64 (dd, 2H), 7.48 (dt, 2H), 7.28 (s, 1H), 7.18 (s, 1H), 3.16-3.09 (m, 2H), 2.28-2.20 (m, 3H), 2.14-2.11 (m, 1H), 2.09-1.93 (m, 4H), 1.89-1.86 (d, 1H), 1.69-1.59 (m, 3H).

### Example 46 (comparative)

### N-(2-fluorophenyl)-7-(6-fluoroquinoline-4-yl) spiro [3.5] nonane-2-carboxamide

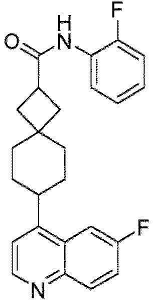

Using 2-fluoroaniline and the product of Step 2 of Example **25,** the title product was prepared by the same method as described in Step 3 for Example **25.**

¹H NMR (400 MHz, CDCl₃) :δ 8.81 (d, 1H), 8.39 (t, 1H), 8.12 (dd, 1H), 7.65 (d, 1H), 7.47 (t, 1H), 7.32-7.26 (m, 2H), 7.17- 6.99 (m, 3H), 3.23-3.06 (m, 2H), 2.25 (dd, 3H), 2.15-1.82 (m, 6H), 1.67 (dd, 3H).

MS ESI: m/z =407.1, [M+H] ⁺.

### Example 47 (comparative)

### N-(2-bromophenyl)-7-(6-fluoroquinoline-4-yl) spiro [3.5] nonane-2-carboxamide

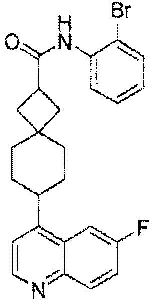

Using 2-bromaniline and the product of Step 2 of Example **25,** the title product was prepared by the same method as described for Example **27.**

¹H NMR (400 MHz, CDCl₃):δ 8.81 (d, 1H), 8.42 (d, 1H), 8.12 (dd, 1H), 7.70-7.58 (m, 2H), 7.54 (dd, 1H), 7.50-7.42 (m, 1H), 7.37-7.30 (m, 1H), 7.28 (d, 1H), 6.97 (dd, 1H), 3.28-3.06 (m, 2H), 2.33-2.21 (m, 3H), 2.02 (m, 6H), 1.74-1.62 (m, 3H).

MS ESI: m/z =467.0, [M+H] ⁺.

### Example 48 (comparative)

### 7-(6-fluoroquinoline-4-yl)-N (pyridine-4-yl) spiro [3.5] nonane-2-carboxamide

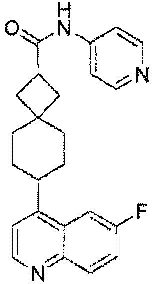

Using 4-aminopyridine and the product of Step 2 of Example **25,** the title product was prepared by the same method as described for Example **27.**

¹H NMR (400 MHz, CDCl₃) δ 8.81 (d, 1H), 8.51 (s, 2H), 8.12 (dd, 1H), 7.65 (dd, 1H), 7.50 (d, 1H), 7.49 (s, 1H), 7.48-7.44 (m, 1H), 7.35 (s, 1H), 7.27 (d, 1H), 3.16 (dd, 2H), 2.29-2.18 (m, 3H), 2.15-1.82 (m, 6H), 1.64 (s, 3H).

MS ESI: m/z =390.1, [M+H] ⁺.

### Example 49 (comparative)

### N-(4-cyanophenyl)-7-(6-fluoroquinolin-4-yl)spiro[3.5]nonane-2-carboxamide

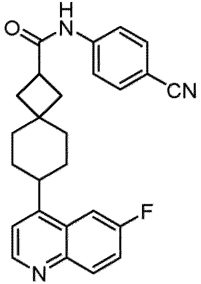

Using 4-aminobenzonitrile and the product of Step 2 of Example **25,** the title product was prepared by the same method as described for Example **27.**

MS ESI: M/Z=414.1, [M+1] ⁺.

¹H NMR (400 MHz, CDCl₃): δ 8.81 (d, 1H), 8.12 (dd, 1H), 7.72-7.59 (m, 4H), 7.51-7.43 (m, 1H), 7.27 (d, 2H), 7.24 (s, 1H), 3.14 (dd, 2H), 2.29-2.19 (m, 3H), 2.16-2.07 (m, 1H), 2.04 (s, 1H), 1.95 (dd, 2H), 1.87 (d, 1H), 1.74-1.63 (m, 4H).

### Example 50 (comparative)

### 7-(6-fluoroquinoline-4-yl)-N-phenyl spiro [3.5] nonane-2-carboxamide

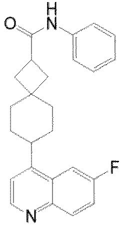

Using aniline and the product of Step 2 of Example **25,** the title product was prepared by the same method as described in Step 3 for Example **25.**

MS ESI: m/z =389.1, [M+H] ⁺.

¹H NMR (400 MHz, CDCl₃):6 8.80(d, 1H), 8.13-8.10(dd, 1H), 7.67-7.64(dd, 1H), 7.54(d, 2H), 7.49-7.44(td, 1H), 7.35-7.31(t, 2H), 7.28-7.27(m, 1H), 7.13-7.09(t, 1H), 7.04(s, 1H), 3.17-3.08(m, 2H), 2.28-2.21(m, 3H), 2.12-2.08(m, 1H), 2.05-2.00(m, 2H), 1.96-1.92(m, 1H), 1.87-1.84(m, 1H), 1.71-1.59(m, 4H).

### Example 51 (comparative)

### N-(4-fluorophenyl)-2-(6-fluoroquinoline-4-yl)-7-azaspiro [3.5] nonane-7-carboxamide

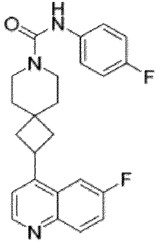

### Step 1: tert-butyl 2-(6-fluoroquinolin-4-yl)-2-hydroxy-7-azaspiro[3.5]nonane-7-carboxylate

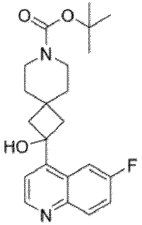

To a solution of 4-bromine-6-fluoroquinoline (500.0 mg, 2.21 mmol) in THF (44 mL) cooled to -78°C was added t-butyllithium solution (2.76 mL, 1.6 M in pentane, 4.42 mmol) slowly under argon. After stirring for 3 min, a solution of 2-oxo-7-azaspiro [3.5] nonane-7-carboxylate (528.8 mg, 2.21 mmol) in 4 mL of THF was added. After stirring at -78 °C for 30min, acetic acid (133 mg) was added and the mixture was concentrated. The residual was purified by silica gel column chromatography eluted with a gradient of 0-100% ethyl acetate/n-hexane to obtain the title product as a light yellow solid (210 mg; yield: 25%).

MS ESI: m/z =387.1, [M+H] ⁺.

¹H NMR (400 MHz, CDCl₃):δ 8.86 (d, 1H), 8.16-8.13(dd, 1H), 7.87-7.85(dd, 1H), 7.52-7.49(m, 1H), 7.49(d, 1H), 3.75-3.70(m, 2H), 3.45-3.43(m, 2H), 3.28-3.26(m, 2H), 2.70-2.51(m, 4H), 2.24-2.20(m, 1H), 1.91-1.88(m, 2H), 1.45(s, 9H).

### Step 2: 6-fluoro-4-(7-Azaspiro [3.5] nonane-2-yl) quinoline

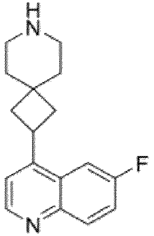

The product (210 mg, 0.544 mmol) of Step 1 was placed into a flask, followed by addition of concentrate hydroiodic acid (2.0 mL, 55%) and red phosphorus (84.0 mg, 2.72 mmol). After the mixture was stirred at 140°C) for 5h, 1 N sodium hydroxide aqueous solution was added to adjust the pH value to 10 and the mixture was treated with Sodium sulfate aqueous solution (20.0 mL). After stirring for 15 min, the solution was extracted with dichloromethane (30.0 mL x 3). The combined organic phase was dried with anhydrous sodium sulfate, filtered and concentrated to obtain the crude product (140 mg; yield: 95%).

MS ESI: m/z =271.1, [M+H]⁺.

¹H NMR (400 MHz, CDCl₃): δ 8.82(d, 1H), 8.13-8.09(m, 1H), 7.48-7.44(m, 2H), 7.28-7.27(m, 1H), 4.03-3.98(m, 1H), 3.66(s, 1H), 3.01-2.98(m, 2H), 2.84-2.81(m, 2H), 2.54-2.49(m, 2H), 2.10-2.05(m, 2H), 1.94-1.91(m, 2H), 1.63-1.60(m, 2H).

### Step 3 N (4-fluorophenyl)-2-(6-fluoroquinoline-4-yl)-7-azaspiro [3.5] nonane-7-carboxamide

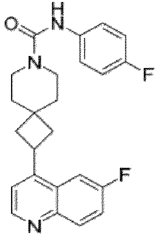

Using 4-fluorophenyl isocyanate and the product of Step 2, the title product was prepared by the same method as described for Example **9.**

MS ESI: m/z =408.1, [M+H] ⁺.

### Example 52 (comparative)

### N-(4-chlorophenyl)-2-(6-fluoroquinoline-4-yl)-7-azaspiro [3.5] nonane-7-carboxamide

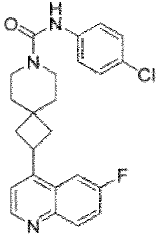

Using 4-chlorophenyl isocyanate and the product of Step 2 of Example 51, the title product was prepared by the same method as described for Example **9.**

MS ESI: m/z =424.1, [M+H] ⁺.

### Example 53 (comparative)

### N-(4-bromophenyl)-2-(6-fluoroquinoline-4-yl)-7-aza-spiro [3.5] nonane-7-carboxamide

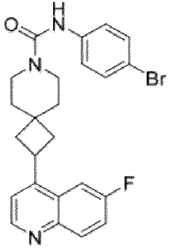

Using 4-bromophenyl isocyanate and the product of Step 2 of Example 51, the title product was prepared by the same method as described for Example **9.**

MS ESI: m/z =468.1, 470.1 [M+H] ⁺.

### Example 54 (comparative)

### N-(4-cyanophenyl)-2-(6-fluoroquinoline-4-yl)-7- azaspiro [3.5] nonane-7-carboxamide

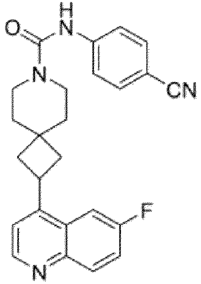

Using 4-isocyanatobenzonitrile isocyanate and the product of Step 2 of Example 51, the title product was prepared by the same method as described for Example **9.**

MS ESI: m/z =415.1, [M+H] ⁺.

### Example 55

### N-(4-chlorophenyl)-7-(6-fluoroquinoline-4-yl)-2-azaspiro [3.5] nonane-2-carboxamide

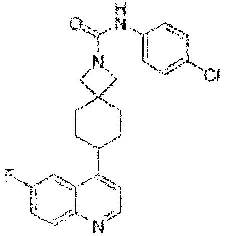

### Step 1: (6-fluoroquinoline-4-yl) boric acid

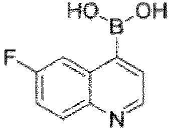

To a solution of 4-bromine-6-fluoroquinoline (1.00 g, 4.42 mmol) and triisopropyl borate (3.06 mL, 13.25 mmol) in 20 mL of THF cooled to -78°C was added t-butyllithium (5.53 mL, 1.6 M in heptane, 8.84 mmol) under N₂. After stirring for 0.5h, saturated ammonium chloride solution (20 mL) was added, and the mixture was extracted with 20 mL x 3 of ethyl acetate. The combined organic phase was washed with brine (20 mL), dried with sodium sulfate, filtered and concentrated to obtain 1.00 g of crude product which was washed with petroleum ether : ethyl acetate (1: 1) to obtain the title compound as a yellow solid (400 mg; yield: 47.4%).

MS ESI: m/z =192.1, [M+H] ⁺.

### Step 2: tert-butyl 7-(((trifluoromethyl)sulfonyl)oxy)-2-azaspiro[3.5]non-6-ene-2-carboxylate

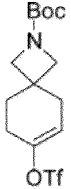

Using the product of Step 1, the title product was prepared by the same method as described in Step 4 for Example **2.**

¹H NMR (400 MHz, CDCl₃): δ 5.67-5.73 (m, 1H), 3.70 (d, 2H), 3.64 (d, 2H), 2.36-2.46 (m, 4H), 1.93-1.98 (m, 2H), 1.44 (s, 9H).

### Step 3: tert-butyl 7-(6-fluoroquinolin-4-yl)-2-azaspiro[3.5]non-6-ene-2-carboxylate

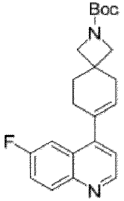

Using the product of Step 2, the title product was prepared by the same method as described in Step 5 for Example **2.**

MS ESI: m/z =369.1, [M+H] ⁺.

### Step 4: tert-butyl 7-(6-fluoroquinolin-4-yl)-2-azaspiro[3.5]nonane-2-carboxylate

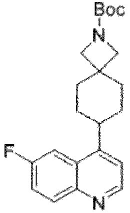

Using the product of Step 3, the title product was prepared by the same method as described in Step 6 for Example **2.**

MS ESI: m/z =371.1, [M+H] ⁺.

### Step 5: 6-fluoro-4-(2-azaspiro[3.5]nonan-7-yl)quinoline di-trifluoroacetate

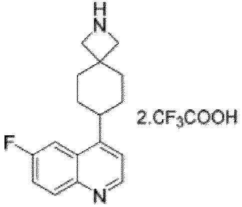

To a solution of the product (120 mg, 0.32 mmol) of Step 4 in 5 mL of dichloromethane was added 1mL of 2,2,2-trifluoroacetic acid, After stirring for 0.5h, the solution was concentrated to obtain the target product as a yellow solid.

MS ESI: m/z =271.1, [M+H] ⁺.

### Step 6: N-(4-chlorophenyl)-7-(6-fluoroquinoline-4-yl)-2-azaspiro [3.5] nonane-2-carboxamide

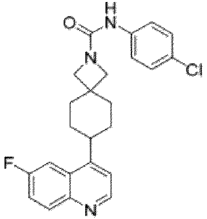

The product (40 mg, 0.08 mmol) of Step 5, triethylamine (0.039 mL, 0.28 mmol) and 2 mL of dichloromethane were placed in a flask. 4-Chlorophenyl isocyanate (15 mg, 0.096 mmol) was added and the mixture was stirred at room temperature for 1h, and concentrated. The residual was purified by silica gel column chromatography to obtain the target product. MS ESI: m/z =424.1, [M+H] ⁺.

### Example 56

### N-(4-bromophenyl)-7-(6-fluoroquinoline-4-yl)-2-azaspiro [3.5] nonane-2-carboxamide

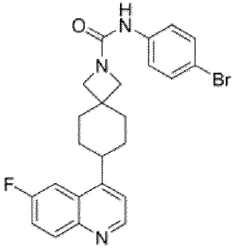

Using 4-bromophenyl isocyanate and the product of Step 5 of Example **55,** the title product was prepared by the same method as described in Step 6 for Example **55.**

MS ESI: m/z =468.0, 470.0, [M+H] ⁺.

### Example 57

### N-(4-cyanophenyl)-7-(6-fluoroquinoline-4-yl)-2-Azaspiro [3.5] nonane-2-carboxamide

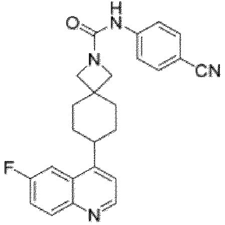

Using 4-cyanophenylisocyanate and the product of Step 5 of Example **55,** the title product was prepared by the same method as described in Step 6 for Example 55.

MS ESI: m/z =415.1, [M+H] ⁺.

### Example 58

### N-(4-fluorophenyl)-7-(6-fluoroquinoline-4-yl)-2-azaspiro [3.5] nonane-2-carboxamide

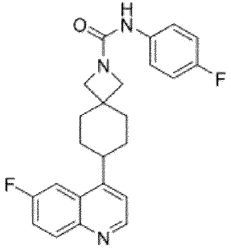

Using 4-fluorophenyl isocyanate and the product of Step 5 of Example 55, the title product was prepared by the same method as described in Step 6 for Example 55.

MS ESI: m/z =408.1, [M+H] ⁺.

### Example 59 Activity test

### (1) Induced expression and purification method of IDO protein

Firstly, DO gene was amplified with PCR, and the amplified PCR product was recycled. Digestion (2h under 37°C), gel running and recycling were carried out on the pET28a plasmid (purchased from Shanghai Baomanbio Co., Ltd.) and IDO gel with two restriction enzymes (EcoR I and Xho I). T4 ligase was connected with the product overnight, added into DH5 α competence, placed on the ice for 30min and subjected to thermal shock at 42°C for 90s. Bacteria coated plate was shaken to pick up monoclonal for PCR identification and sequencing; if all were accurate, it indicated that pET28a-IDO plasmid was established successfully.

BL21 which contained pET28a-IDO plasmid was shaken vigorously at 37°C till OD₆₀₀ was 0.6-0.8, added into hemin (final concentration: 40µM) and 0.5mM IPTG (isopropyl- β -D-IPTG) and induced under 16°C for 20h; after induction, thallus were collected centrifugally at 4°C and 6,000rpm, washed with 50mM PBS (pH 7.5) and collected again centrifugally.

The collected thallus were suspended again with buffer solution (50mM PBS pH 7.5). An appropriate amount of 100 x PMSF was added. The thallus were disrupted with cell disruption instrument (1,400bar) at 4°C for three times. The split bacteria were centrifuged at 18,000 x g for 45min. The sediment was removed but supernatant was retained and filtered with a 0.45µm film at 4°C; the nickel column was balanced with lysis buffer (50mM PBS pH7.5) by 3 column volumes. The supernatant was loaded on the nickel column and washed with wash solution (50mM PBS pH7.5, 50mM imidazole) by 4 column volume. Finally, protein was eluted with eluant (50mM PBS pH7.5, 250mM imidazole); dialyzed for 6h with dialysis solution (50mM PBS pH7.5), concentrated, sub-packaged, quickly frozen with liquid nitrogen and stored at -80°C for backup.

### (2) Test method for IDO enzyme inhibitory activity

Firstly, the compound was diluted by triple gradient. 1µL of each concentration was added into a 96-well plate; 50µL of IDO Enzyme solution PBS (pH 7.5; final concentration: 50mM) was added: 25µL of substrate 1 (methylene blue, final concentration: 3.5M; catalase, final concentration: 0.2µg/L; PBS (pH 7.5; final concentration: 50 mM) mixture and 25µL of substrate 2 (D-Trp, final concentration: 1.5mM; sodium ascorbate, final concentration: 20mM; PBS (pH 7.5), final concentration 50mM) mixture were added for starting the reaction. Finally, OD₃₂₁ nm reading was carried out for 40min.

### (3) Test method of cellular activity

Hela cells (80µE) was inoculated on a 96-well plate (5 x 10³ for each well) to grow overnight. After the compound was diluted the next day, 1µL of diluent and 100µL of culture medium which contains human interferon γ (final concentration: 50ng/mL) were added into the 96-well plate to make the final volume reach 200µL. After incubating for 48h, 80µL of supernate was transferred from each well to a new 3894-well plate. 10µL 6. 1N trichloroacetic acid was added into each well for mixing and incubation at 50 °C for 30min, and IDO catalyzed N formyl-kynurenin into kynurenine. The reaction mixture was centrifuged at 2,500rpm for 10min, 70µL of supernatant of each well was transferred to a new 96-well plate and mixed with 100µL of 2% (w/v) dimethylaminobenzaldehyde acetic acid solution. After placing for 5-10min, measurement was carried out at 480 nm with the SPECTRAmax i3 reader.

The test result of IDO enzyme and celllular inhibitory activity of compounds is shown as Table 1.

**Table 1 Test Result of IDO Enzyme and Cellular Inhibitory Activity**

| Compound number | IDO enzyme inhibitory activity IC₅₀ (µM) | IPS cells IC₅₀ (nM) |
|---|---|---|
| **1*** | 1.1 | >50 |
| **2A*** | 1.3 | >50 |
| **2B*** | 0.5 | >50 |
| **3*** | 0.63 | >3 |
| **4*** | 0.16 | 22 |
| **5*** | 0.33 | >3 |
| **6*** | 0.47 | >4 |
| **7*** | 0.99 | |
| **8*** | 0.99 | >30 |
| **9*** | 3.0 | |
| **10A*** | 0.52 | >3 |
| **10B*** | 2.1 | >3 |
| **11A*** | 6.9 | |
| **11B*** | 2.7 | |
| **12A*** | 1.2 | |
| **12B*** | 1.18 | >30 |
| **13A*** | 20 | |
| **13B*** | 157 | |
| **14A*** | 7.6 | |
| **14B*** | 7 | |
| **15A*** | 3.66 | >10 |
| **15B*** | 0.4 | 2.53 |
| **16*** | 3.2 | |
| **17*** | 0.48 | |
| **18*** | 0.20 | 2.52 |
| **19** | 0.40 | 3.36 |
| **20*** | 2.42 | >10 |
| **21*** | 0.48 | >10 |
| **22A*** | 4.16 | |
| **22B*** | 3.44 | |
| **23*** | | |
| **24*** | | |
| **25** | | 0.69 |
| **26*** | | 0.79 |
| **27*** | | >10 |
| **28*** | | >10 |
| **29*** | | >10 |
| **30*** | | 2.53 |
| **31*** | | 1.97 |
| **32*** | | >10 |
| **33*** | | >10 |
| **34*** | | 1.36 |
| **35*** | | 6.23 |
| **36*** | | 8.32 |
| **37*** | | >10 |
| **38*** | | >10 |
| **39*** | | >10 |
| **40*** | | 3.05 |
| **41*** | | >10 |
| **42*** | | 9.50 |
| **43*** | | 3.07 |
| **44*** | | >10 |
| **45*** | | >10 |
| **46*** | | 2.74 |
| **47*** | | >10 |
| **48*** | | >10 |
| **49*** | | 1.32 |

| | | |
|---|---|---|
| * = comparative | | |

The above result shows that the compound in the invention has inhibitory activity for IDO enzyme and cell.

All the literature reviews in the invention were used as the reference of the application, just like individual reference of each literature review. Besides, it's noteworthy that, after reading the above content, the technicians in this field can change or modify the invention.

## Claims

1. A compound selected from the group of
*N*-(4-fluorophenyl)-7-(6-fluoroquinolin-4-yl)spiro[3.5]nonane-2-carboxamide,
*N*-(4-chlorophenyl)-7-(6-fluoroquinoline-4-yl)-2-azaspiro [3.5] nonane-2-carboxamide,
*N*-(4-bromophenyl)-7-(6-fluoroquinoline-4-yl)-2-azaspiro [3.5] nonane-2-carboxamide,
*N*-(4-cyanophenyl)-7-(6-fluoroquinoline-4-yl)-2-azaspiro [3.5] nonane-2-carboxamide, and
*N*-(4-fluorophenyl)-7-(6-fluoroquinoline-4-yl)-2-azaspiro [3.5] nonane-2-carboxamide
or their stereoisomers or tautomers, or pharmaceutically-acceptable salts.

2. The compound of claim 1, wherein the compound is *N*-(4-fluorophenyl)-7-(6-fluoroquinolin-4-yl)spiro[3.5]nonane-2-carboxamide or its stereoisomer or tautomer, or pharmaceutically-acceptable salt.

3. The *N*-(4-fluorophenyl)-7-(6-fluoroquinoline-4-yl) spiro [3.5] nonane-2-carboxamide of claim 2 as a pure enantiomer obtainable from
(S)-1-phenylethyl (±)7-(6-fluoroquinoline-4-yl) spiro [3.5] nonane-2-carboxylic ester by a method comprising the steps
- Separation performed with Agilent 1260 semi-preparative liquid chromatograph system on a Chiral DAICEL IC 30 x 250 mm column, 5 µm at a Flow rate of 1.0 mL/min and the Eluent: n-hexane: Ethanol=80: 20 (volume ratio), using a Detection wavelength of 254 nm and collecting two peaks at 10.89 min (isomer A) and 12.52 min (isomer B), wherein
- 2.98 g Isomer A is dissolved in 5.0 mL methanol under N2,
- 70 mg palladium hydroxide carbon catalyst (10%) and pure water (0.08 mL) are added and the mixture is stirred at room temperature under H2 for 16h
- the Palladium catalyst is filtered off through celite
- the filtrate is concentrated and the residual is purified by silica gel column chromatography for purification and eluted with a gradient of 0-10% methanol/ dichloromethane to obtain a product
- 30 mg of the product, 24 mg 4-fluoroaniline, 37 mg 1-ethyl-(3-dimethyllaminopropyl) carbonyl diamide hydrochloride and 19 mg 1-hydroxybenzotriazole are added into 5.0 mL dichloromethane.
- 29 mg triethylamine is added
- the mixture is stirred at room temperature for 16 h in an ice bath
- pouring the reaction mixture was poured into 10 mL water and extracting with 30 mL ethyl acetate, followed by washing the organic phase twice with 20 ml saturated sodium bicarbonate, then with 20 ml brine and drying with anhydrous sodium sulfate
- filtration, concentration and purification by silica gel column chromatography eluted with a gradient of 0-60% ethyl acetate/n-hexane.

4. The compound of claim 1, wherein the compound is *N*-(4-chlorophenyl)-7-(6-fluoroquinoline-4-yl)-2-azaspiro [3.5] nonane-2-carboxamide or its stereoisomer or tautomer, or pharmaceutically-acceptable salt.

5. The compound of claim 1, wherein the compound is *N*-(4-bromophenyl)-7-(6-fluoroquinoline-4-yl)-2-azaspiro [3.5] nonane-2-carboxamide or its stereoisomer or tautomer, or pharmaceutically-acceptable salt.

6. The compound of claim 1, wherein the compound is *N*-(4-cyanophenyl)-7-(6-fluoroquinoline-4-yl)-2-Azaspiro [3.5] nonane-2-carboxamide or its stereoisomer or tautomer, or pharmaceutically-acceptable salt.

7. The compound of claim 1, wherein the compound is *N*-(4-fluorophenyl)-7-(6-fluoroquinoline-4-yl)-2-azaspiro [3.5] nonane-2-carboxamide or its stereoisomer or tautomer, or pharmaceutically-acceptable salt.

8. The compounds of claim 1 to 7 or their stereoisomers or tautomers, or pharmaceutically-acceptable salts wherein the pharmaceutically acceptable salts are selected from the following groups: Hydrochloride, hydrobromate, sulfate, phosphate, mesylate, trifluoromethylsulfonate, benzene sulfonate, p-toluenesulfonate (tosylate), 1-Naphthalenesulfonate, 2-Naphthalenesulfonate, acetate, trifluoroacetate, malate, tartrate, citrate, lactate, oxalate, succinate, fumarate, maleate, benzoate, salicylate, phenylacetate and mandelate.

9. The compounds of claim 1 to 7 or their stereoisomers or tautomers, or pharmaceutically-acceptable salts for use as an antitumor drug.

10. The compounds of claim 1 to 7 or their stereoisomers or tautomers, or pharmaceutically-acceptable salts for use in the prevention and/or treatment of indoleamine-2, 3-dioxygenase mediated diseases **characterized in that** the indoleamine-2,3-dioxygenase mediated diseases are selected from cancer, neurodegenerative disease, HIV infections, eye diseases, psychological disorders, depression, anxiety disorder, Alzheimer and/or autoimmune disease.

11. A pharmaceutical composition comprising compounds of claim 1 to 7 or their stereoisomers or tautomers, or pharmaceutically-acceptable salts, a pharmaceutically acceptable carriers and other antitumor drugs, such as chemotherapy drugs, targeted antitumor drugs and check-point protein antibodies.

## Patentansprüche

1. Verbindung, ausgewählt aus der Gruppe:
N-(4-Fluorphenyl)-7-(6-fluorchinolin-4-yl)spiro[3.5]nonan-2-carboxamid,
N-(4-Chlorphenyl)-7-(6-fluorchinolin-4-yl)-2-azaspiro[3.5]nonan-2-carboxamid,
N-(4-Bromphenyl)-7-(6-fluorchinolin-4-yl)-2-azaspiro[3.5]nonan-2-carboxamid,
N-(4-Cyanophenyl)-7-(6-fluorchinolin-4-yl)-2-azaspiro[3.5]nonan-2-carboxamid und
N-(4-Fluorphenyl)-7-(6-fluorchinolin-4-yl)-2-azaspiro[3.5]nonan-2-carboxamid
oder deren Stereoisomeren oder Tautomeren oder pharmazeutisch annehmbaren Salzen.

2. Verbindung gemäß Anspruch 1, wobei es sich bei der Verbindung um
*N*-(4-Fluorphenyl)-7-(6-fluorchinolin-4-yl)spiro[3.5]nonan-2-carboxamid oder dessen Stereoisomer oder Tautomer oder pharmazeutisch annehmbares Salz handelt.

3. N-(4-Fluorphenyl)-7-(6-fluorchinolin-4-yl)spiro[3.5]nonan-2-carboxamid gemäß Anspruch 2 als reines Enantiomer, erhältlich aus
(S)-1-Phenylethyl-(±)7-(6-fluorchinolin-4-yl)spiro[3.5]nonan-2-carbonsäureester nach einem Verfahren, das die folgenden Schritte umfasst:
- Trennung durchgeführt mit Agilent 1260 semipräparativem Flüssigchromatographsystem auf einer Säule des Typs Chiral DAICEL IC 30 x 250 mm, 5 µm mit einer Fließgeschwindigkeit von 1,0 ml/min und einem Volumenverhältnis von Eluent:n-Hexan:Ethanol = 80:20 unter Verwendung einer Nachweiswellenlänge von 254 nm und Auffangen bei zwei Maxima nach 10,89 min (Isomer A) und 12,52 min (Isomer B), wobei
- 2,98 g Isomer A unter N₂ in 5,0 ml Methanol gelöst wird,
- 70 mg Palladiumhydroxid/Kohle-Katalysator (10%) und reines Wasser (0,08 ml) hinzugefügt werden und das Gemisch 16 h lang unter H₂ bei Raumtemperatur gerührt wird,
- der Palladium-Katalysator über Celite abfiltriert wird,
- das Filtrat eingeengt wird und der Rückstand durch Silicagel-Säulenchromatographie gereinigt und mit einem Gradienten von 0-10% Methanol/Dichlormethan eluiert wird, wobei man ein Produkt erhält,
- 30 mg des Produkts, 24 mg 4-Fluoranilin, 37 mg 1-Ethyl-(3-dimethylaminopropyl)carbonyldiamid-Hydrochlorid und 19 mg 1-Hydroxybenzotriazol zu 5,0 ml Dichlormethan gegeben werden,
- 29 mg Triethylamin hinzugefügt wird,
- das Gemisch 16 h lang bei Raumtemperatur in einem Eisbad gerührt wird,
- das Reaktionsgemisch in 10 ml Wasser gegossen und mit 30 ml Ethylacetat extrahiert wird, anschließend die organische Phase zweimal mit 20 ml gesättigter Natriumhydrogencarbonatlösung, dann mit 20 ml Kochsalzlösung gewaschen und mit wasserfreiem Natriumsulfat getrocknet wird,
- Filtration, Einengen und Reinigen durch Silicagel-Säulenchromatographie, eluiert mit einem Gradienten von 0-60% Ethylacetat/n-Hexan.

4. Verbindung gemäß Anspruch 1, wobei es sich bei der Verbindung um
N-(4-Chlorphenyl)-7-(6-fluorchinolin-4-yl)-2-azaspiro[3.5]nonan-2-carb-oxamid oder dessen Stereoisomer oder Tautomer oder pharmazeutisch annehmbares Salz handelt.

5. Verbindung gemäß Anspruch 1, wobei es sich bei der Verbindung um
N-(4-Bromphenyl)-7-(6-fluorchinolin-4-yl)-2-azaspiro[3.5]nonan-2-carb-oxamid oder dessen Stereoisomer oder Tautomer oder pharmazeutisch annehmbares Salz handelt.

6. Verbindung gemäß Anspruch 1, wobei es sich bei der Verbindung um
N-(4-Cyanophenyl)-7-(6-fluorchinolin-4-yl)-2-azaspiro[3.5]nonan-2-carb-oxamid oder dessen Stereoisomer oder Tautomer oder pharmazeutisch annehmbares Salz handelt.

7. Verbindung gemäß Anspruch 1, wobei es sich bei der Verbindung um
N-(4-Fluorphenyl)-7-(6-fluorchinolin-4-yl)-2-azaspiro[3.5]nonan-2-carbox-amid oder dessen Stereoisomer oder Tautomer oder pharmazeutisch annehmbares Salz handelt.

8. Verbindungen gemäß den Ansprüchen 1 bis 7 oder ihre Stereoisomere oder Tautomere oder pharmazeutisch annehmbaren Salze, wobei die pharmazeutisch annehmbaren Salze aus den folgenden Gruppen ausgewählt sind: Hydrochlorid, Hydrobromat, Sulfat, Phosphat, Mesylat, Trifluormethylsulfonat, Benzolsulfonat, p-Toluolsulfonat (Tosylat), 1-Naphthalinsulfonat, 2-Naphthalinsulfonat, Acetat, Trifluoracetat, Malat, Tartrat, Citrat, Lactat, Oxalat, Succinat, Fumarat, Maleat, Benzoat, Salicylat, Phenylacetat und Mandelat.

9. Verbindungen gemäß Anspruch 1 bis 7 oder ihre Stereoisomere oder Tautomere oder pharmazeutisch annehmbaren Salze zur Verwendung als Antitumorwirkstoff.

10. Verbindungen gemäß Anspruch 1 bis 7 oder ihre Stereoisomere oder Tautomere oder pharmazeutisch annehmbaren Salze zur Verwendung bei der Prävention und/oder Behandlung von Erkrankungen, die durch Indolamin-2,3-dioxygenase vermittelt werden, **dadurch gekennzeichnet, dass** die durch Indolamin-2,3-dioxygenase vermittelten Erkrankungen aus Krebs, neurodegenerativer Erkrankung, HIV-Infektionen, Augenerkrankungen, psychologischen Störungen, Depressionen, Angststörungen, Alzheimer-Erkrankung und/oder Autoimmunerkrankung ausgewählt sind.

11. Pharmazeutische Zusammensetzung, umfassend Verbindungen gemäß Anspruch 1 bis 7 oder ihre Stereoisomere oder Tautomere oder pharmazeutisch annehmbaren Salze, pharmazeutisch annehmbare Träger und andere Antitumorwirkstoffe, wie Chemotherapeutika, zielgesteuerte Antitumorwirkstoffe und Checkpoint-Protein-Antikörper.

## Revendications

1. Composé choisi dans le groupe suivant :
le *N*-(4-fluorophényl)-7-(6-fluoroquinoléin-4-yl)spiro[3.5]nonane-2-carboxamide,
le *N*-(4-chlorophényl)-7-(6-fluoroquinoléine-4-yl)-2-azaspiro[3.5]nonane-2-carboxamide,
le *N*-(4-bromophényl)-7-(6-fluoroquinoléine-4-yl)-2-azaspiro[3.5]nonane-2-carboxamide,
le *N*-(4-cyanophényl)-7-(6-fluoroquinoléine-4-yl)-2-azaspiro[3.5]nonane-2-carboxamide, et
le *N*-(4-fluorophényl)-7-(6-fluoroquinoléine-4-yl)-2-azaspiro[3.5]nonane-2-carboxamide
ou leurs stéréoisomères ou tautomères, ou leurs sels pharmaceutiquement acceptables.

2. Composé selon la revendication 1, le composé étant le *N*-(4-fluorophényl)-7-(6-fluoroquinoléin-4-yl)spiro[3.5]nonane-2-carboxamide ou son stéréoisomère ou tautomère, ou son sel pharmaceutiquement acceptable.

3. *N*-(4-fluorophényl)-7-(6-fluoroquinoléine-4-yl)spiro[3.5]nonane-2-carboxamide selon la revendication 2 en tant qu'énantiomère pur pouvant être obtenu à partir de l'ester (S)-1-phényléthyl(±)7-(6-fluoroquinoléine-4-yl)spiro[3.5]nonane-2-carboxylique par un procédé comprenant les étapes suivantes :
- séparation effectuée avec un système de chromatographie liquide semi-préparative Agilent 1260 sur une colonne chirale DAICEL IC 30 x 250 mm, 5 µm à un débit de 1,0 mL/min et l'éluant : n-hexane:éthanol = 80:20 (rapport de volume), en utilisant une longueur d'onde de détection de 254 nm et en collectant deux pics à 10,89 min (isomère A) et 12,52 min (isomère B), dans lequel
- 2,98 g d'isomère A sont dissous dans 5,0 mL de méthanol sous N₂,
- 70 mg de catalyseur d'hydroxyde de palladium carbone (10 %) et de l'eau pure (0,08 mL) sont ajoutés et le mélange est agité à température ambiante sous H₂ pendant 16 h
- le catalyseur de palladium est éliminé par filtration sur de la célite
- le filtrat est concentré et le résidu est purifié par chromatographie sur colonne de gel de silice pour purification et élué avec un gradient de 0 à 10 % de méthanol/dichlorométhane pour obtenir un produit
- 30 mg du produit, 24 mg de 4-fluoroaniline, 37 mg de chlorhydrate de 1-éthyl-(3-diméthyllaminopropyl)carbonyldiamide et 19 mg de 1-hydroxybenzotriazole sont ajoutés dans 5,0 mL de dichlorométhane
- 29 mg de triéthylamine sont ajoutés
- le mélange est agité à température ambiante pendant 16 h dans un bain de glace
- versage du mélange de réaction qui a été versé dans 10 mL d'eau et extraction avec 30 mL d'acétate d'éthyle, suivie d'un double lavage de la phase organique avec 20 mL de bicarbonate de sodium saturé, puis avec 20 mL de saumure et d'un séchage avec du sulfate de sodium anhydre
- filtration, concentration et purification par chromatographie sur colonne de gel de silice éluée avec un gradient de 0 à 60 % d'acétate d'éthyle/n-hexane.

4. Composé selon la revendication 1, le composé étant
le *N*-(4-chlorophényl)-7-(6-fluoroquinoléine-4-yl)-2-azaspiro[3.5]nonane-2-carboxamide
ou son stéréoisomère ou tautomère, ou son sel pharmaceutiquement acceptable.

5. Composé selon la revendication 1, le composé étant
le *N*-(4-bromophényl)-7-(6-fluoroquinoléine-4-yl)-2-azaspiro[3.5]nonane-2-carboxamide
ou son stéréoisomère ou tautomère, ou son sel pharmaceutiquement acceptable.

6. Composé selon la revendication 1, le composé étant
le *N*-(4-cyanophényl)-7-(6-fluoroquinoléine-4-yl)-2-azaspiro[3.5]nonane-2-carboxamide
ou son stéréoisomère ou tautomère, ou son sel pharmaceutiquement acceptable.

7. Composé selon la revendication 1, le composé étant
le *N*-(4-fluorophényl)-7-(6-fluoroquinoléine-4-yl)-2-azaspiro[3.5]nonane-2-carboxamide
ou son stéréoisomère ou tautomère, ou son sel pharmaceutiquement acceptable.

8. Composé selon les revendications 1 à 7 ou leurs stéréoisomères ou tautomères, ou leurs sels pharmaceutiquement acceptables, dans lesquels les sels pharmaceutiquement acceptables sont choisis parmi les groupes suivants : le chlorhydrate, le bromhydrate, le sulfate, le phosphate, le mésylate, le sulfonate de trifluorométhyle, le sulfonate de benzène, le p-toluènesulfonate (tosylate), le 1-naphtalènesulfonate, le 2-naphtalènesulfonate, l'acétate, le trifluoroacétate, le malate, le tartrate, le citrate, le lactate, l'oxalate, le succinate, le fumarate, le maléate, le benzoate, le salicylate, le phénylacétate et le mandélate.

9. Composés selon les revendications 1 à 7 ou leurs stéréoisomères ou tautomères, ou leurs sels pharmaceutiquement acceptables, pour une utilisation comme médicament antitumoral.

10. Composés selon les revendications 1 à 7 ou leurs stéréoisomères ou tautomères, ou leurs sels pharmaceutiquement acceptables, pour une utilisation dans la prévention et/ou le traitement de maladies médiées par l'indoleamine-2,3-dioxygénase **caractérisés en ce que** les maladies médiées par l'indoleamine-2,3-dioxygénase sont choisies parmi le cancer, les maladies neurodégénératives, les infections par le VIH, les maladies oculaires, les troubles psychologiques, la dépression, le trouble anxieux, la maladie d'Alzheimer et/ou les maladies auto-immunes.

11. Composition pharmaceutique comprenant des composés des revendications 1 à 7 ou leurs stéréoisomères ou tautomères, ou leurs sels pharmaceutiquement acceptables, un support pharmaceutiquement acceptable et d'autres médicaments antitumoraux, tels que des médicaments de chimiothérapie, des médicaments antitumoraux ciblés et des anticorps contre les protéines points de contrôle.
